(19) Europäisches Patentamt
European Patent Office
Office européen des brevets

(11) Publication number: **0 313 874 B1**

(12) # EUROPEAN PATENT SPECIFICATION

(45) Date of publication of patent specification: **13.04.94**

(51) Int. Cl.$^5$: **C07H 15/20**, A61K 31/70

(21) Application number: **88116339.8**

(22) Date of filing: **03.10.88**

(54) **Disulfur analogs of LL-E33288 antitumor agents.**

(30) Priority: **30.10.87 US 114940**

(43) Date of publication of application:
**03.05.89 Bulletin 89/18**

(45) Publication of the grant of the patent:
**13.04.94 Bulletin 94/15**

(84) Designated Contracting States:
**AT BE CH DE ES FR GB GR IT LI NL SE**

(56) References cited:
**EP-A- 0 182 152**

**J AM CHEM SOC, vol 109, 1987, pages 3466-3468, American Chemical Society, US; M D Lee etal.: "Calichemicins, a novel family of antitumor antibiotics. 2. Chemistry and structure of calichemicin gamma**

(73) Proprietor: **AMERICAN CYANAMID COMPANY**
**One Cyanamid Plaza**
**Wayne, NJ 07470-8426(US)**

(72) Inventor: **McGahren, William James**
**64 Glenwood Avenue**
**Demarest New Jersey 07627(US)**
Inventor: **Sassiver, Martin Leon**
**6 Hadassah Lane**
**Spring Valley New York 10977(US)**
Inventor: **Ellestad, George A.**
**59 Lt. Cox Drive**
**Pearl River New York 10965(US)**

(74) Representative: **Wächtershäuser, Günter, Dr.**
**Tal 29**
**D-80331 München (DE)**

**EP 0 313 874 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Table 1: Proposed Structures for $CH_3$-SS-W (wherein W is the substituent attached to $CH_3$-SSS- below)

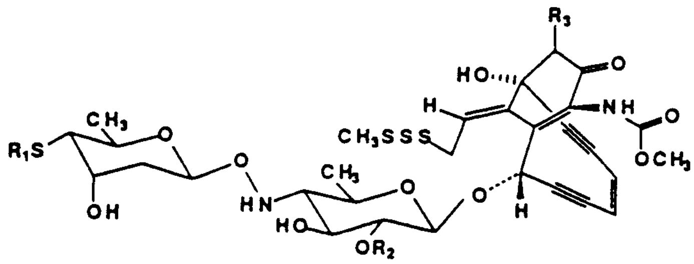

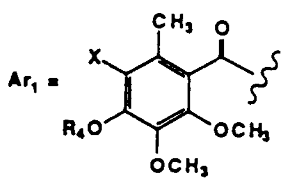

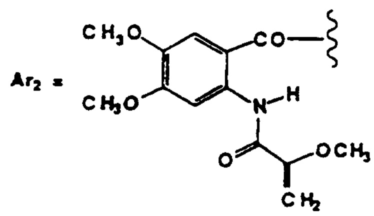

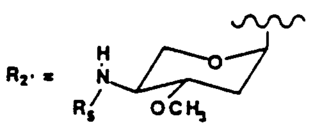

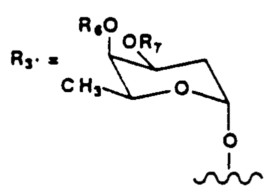

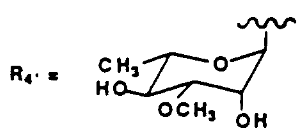

| Designation | $R_1$ | $R_2$ | $R_3$ | $R_4$ | $R_5$ | $R_6$ | $R_7$ | X |
|---|---|---|---|---|---|---|---|---|
| LL-E33288$\alpha_2{}^I$ | $Ar_1$ | $R_{2'}$ | H | H | $C_2H_5$ | | | I |
| LL-E33288$\alpha_3{}^I$ | $Ar_1$ | H | H | $R_{4'}$ | | | | I |
| LL-E33288$\beta_1{}^I$ | $Ar_1$ | $R_{2'}$ | H | $R_{4'}$ | $(CH_3)_2CH$ | | | I |
| LL-E33288$\gamma_1{}^I$ | $Ar_1$ | $R_{2'}$ | H | $R_{4'}$ | $C_2H_5$ | | | I |
| LL-E33288$\delta_1{}^I$ | $Ar_1$ | $R_{2'}$ | H | $R_{4'}$ | $CH_3$ | | | I |
| LL-E33288$\beta_1{}^{Br}$ | $Ar_1$ | $R_{2'}$ | H | $R_{4'}$ | $(CH_3)_2CH$ | | | Br |
| LL-E33288$\gamma_1{}^{Br}$ | $Ar_1$ | $R_{2'}$ | H | $R_{4'}$ | $C_2H_5$ | | | Br |
| LL-E33288$\alpha_2{}^{Br}$ | $Ar_1$ | $R_{2'}$ | H | H | $C_2H_5$ | | | Br |
| LL-E33288$\alpha_3{}^{Br}$ | $Ar_1$ | H | H | $R_{4'}$ | | | | Br |
| Esperamicin $A_1$ | $CH_3$ | $R_{2'}$ | $R_{3'}$ | | $(CH_3)_2CH$ | H | $Ar_2$ | |
| Esperamicin $A_2$ | $CH_3$ | $R_{2'}$ | $R_{3'}$ | | $(CH_3)_2CH$ | $Ar_2$ | H | |
| Esperamicin $A_{1b}$ | $CH_3$ | $R_{2'}$ | $R_{3'}$ | | $CH_3CH_2$ | H | $Ar_2$ | |

Disulfur analogs of the invention are also prepared from certain other antibiotics, namely:

1) Esperamicin BBM-1675, a novel class of potent antitumor. I. Physico-chemical data and partial structure. M. Konishi, et al., J. Antibiotics, 38, 1605 (1985). A new antitumor antibiotic complex. M. Konishi, et al., U.K. Patent Application GB 2,141,425A, May 15, 1984.

2) New antitumor antibiotics, FR-900405 and FR-900406.I. Taxonomy of the producing strain. M. Iwami, et al., J. antibiotics 38, 835 (1985). New antitumor antibiotics FR-900405 and FR-900406.II. Production, isolation, characterization and antitumor activity, S. Kiyoto, et al., J. Antibiotics, 38, 340 (1985).

3) PD 114759 and PD 115028, novel antitumor antibiotics with phenomenal potency. I. Isolation and characterization. R. H. Bunge, et al., J. Antibiotics, 37, 1566 (1984). Biological and biochemical activities of the novel antitumor antibiotic PD 114759 and related derivatives. D. W. Fry et al., Investigational New Drugs, 4, 3 (1986).

4) New antibiotic complex CL-1577A, CL-1577B produced by Streptomyces sp. ATCC 39363. European Patent Application 0,132,082, A2.

5) CL-1577D and CL-1577E Antibiotic antitumor compounds, their production and use. U.S. Patent 4,539,203.

6) CL-1724 Antibiotic compounds, their production and use. U. S. Patent 4,554,162.

All of the information regarding BBM-1675, FR-900405, FR-900406, PD 114759, PD 115028, CL-1577A, CL-1577B, CL-11577D, CL-1577E and CL-1724 contained in the above citations is incorporated herein by reference.

As can be seen from the structures disclosed in Table I, the $\alpha_1$, $\alpha_2$, $\alpha_3$, $\alpha_4$ $\beta_1$, $\beta_2$, $\gamma_1$ and $\delta$components of the LL-E33288 complex, as well as the BBM-1675, FR-900405, FR-900406, PD-114759, PD 115028, CL-1577A, CL-1577B, CL-1577D, CL-1577E and CL-1724 antibiotics each contain an allylic trisulfide group in their structures.

It has now been discovered that the reaction of any of the above cited antibiotics with an unsubstituted or substituted alkyl or aryl mercaptan results in displacement of the methyl perthiolate anion from the trisulfide moiety resulting in the formation of a stable disulfide analog (Scheme I), below.

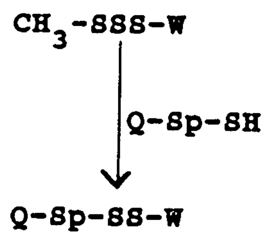


**Scheme I**

The compounds of Table I are transformed with thiol-containing organic molecules according to Scheme I to produce new compositions useful as antibacterial and anticancer agents in their own right, wherein W, $R_1$, $R_2$, $R_3$, $R_4$, $R_5$, $R_6$, $R_7$, $R_2'$, $R_3'$, $R_4'$, $Ar_1$, $Ar_2$ and X are as hereinbefore defined in Table I, Sp is straight or branched-chain divalent($C_1$-$C_{18}$)radicals, divalent aryl or heteroaryl radicals, divalent($C_3$-$C_{18}$)-cycloalkyl radicals, di-valent($C_1$-$C_{18}$)aryl- or heteroaryl-alkyl radicals, divalent($C_1$-$C_{18}$)cycloalkyl- alkyl radicals, or divalent ($C_2$-$C_{18}$) unsaturated alkyl radicals; Q is hydrogen, halogen, amino, alkylamino, dialkylamino, piperidino, pyrrolidino, piperazino, carboxyl, carboxaldehyde, lower alkoxy, hydroxy, thiol, lower alkyldicarboxyl, or a group chosen from the following: $-CONHNH_2$, $-NHCONHNH_2$, $-NHCSNHNH_2$, or $-ONH_2$; with the proviso that when Sp is ethylidine, Q cannot be hydrogen.

As an example, reaction of LL-E33288$\gamma_1$-I with methyl mercaptan in acetonitrile produces LL-E33288$\gamma_2$-I, a new antitumor compound, which is also produced by the aerobic fermentation conditions described in the above cited patent applications.

The above reaction is not limited to the iodo derivatives of the LL-E33288 antibiotics. If, for example, LL-E33288$\gamma_1$-Br is used, the product will be LL-E33288$\gamma_2$-Br and in fact the alkyl disulfide bromo analog of any E33288-bromo antibiotic will be realized under similar conditions.

The proposed structure of the two aforementioned antibiotics after reaction with methyl mercaptan are given below.

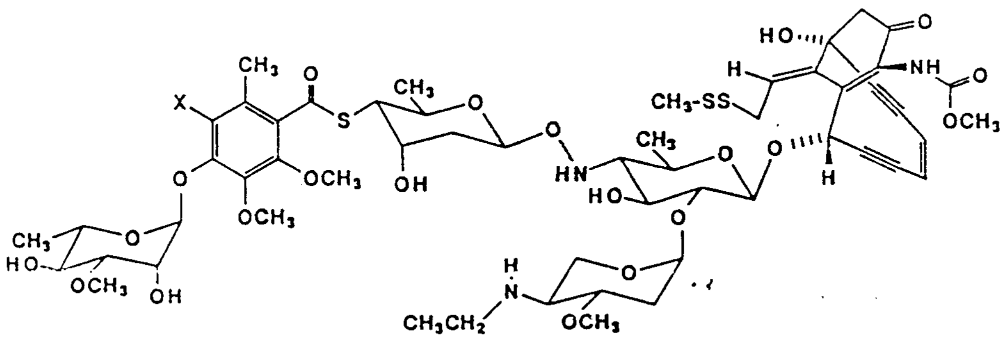

R = $CH_3$, X = I = LL33288$\gamma_2^{I}$
R = $CH_3$, X = Br = LL33288$\gamma_2^{Br}$

The transformation to the disulfur analog is by no means restricted to reactions of the compounds listed in Table I with methyl mercaptan, but can be used with a large variety of thiol-containing organic molecules to yield novel antitumor and antibacterial agents. Moreover, the products from the reaction themselves are amenable to further transformations within the newly-introduced side-chain to produce still more novel compounds with biological activities.

The compounds of this invention in addition to their use as antitumor and antibacterial agents, are useful for assessing new modes of anticancer activity at a cellular level. The naturally-derived antibiotics described by the above-named applications and patents do not contain sufficiently strong chromophoric units such that their intracellular localization can be adequately delineated. When the native trithiomethyl derivatives are reacted with thiol-containing molecules which additionally contain a chromophoric unit that absorbs or emits light in areas of the electromagnetic spectrum not obscured by cellular chromophores, useful biochemical tools for studying double strand DNA breaks and cellular localization of this class of compounds can be generated. Similarly, the introduction of radiolabels by this reaction is within the scope of our invention. Thus, for example, when E332888$\gamma_1$-I is reacted with 7-(2-thioethylamino)-4-methylcoumarin, a derivative is obtained which fluoresces intensely upon irradiation with ultraviolet light, permitting location of the compound within cellular compartments.

In this embodiment of this invention, the compounds of Table I are transformed with thiol-containing organic molecules according to Scheme 1 to produce new compositions additionally useful as molecular probes, wherein W, $R_1$, $R_2$, $R_3$, $R_4$, $R_5$, $R_6$, $R_7$, $R_2'$, $R_3'$, $R_4'$, $Ar_1$, $Ar_2$, and X are as hereinbefore defined in Table I, Sp is straight or branched-chain divalent ($C_1$-$C_{18}$) radicals, divalent aryl or heteroaryl radicals, divalent ($C_3$-$C_{18}$) cycloalkyl radicals, divalent ($C_1$-$C_{18}$) aryl- or heteroaryl-alkyl radicals, divalent ($C_1$-$C_{18}$) cycloalkyl -alkyl radicals, and divalent ($C_2$-$c_{18}$) unsaturated alkyl radicals; Q is unsubstituted or substituted fluorescing nuclei of dibenzooxazine, rhodamine, carbostyril, coumarin, fluorecein, or acridine, or a substituent containing a $^3H$, $^{14}C$, $^{35}S$, or $^{32}P$ radiolabel as part of its structure.

The compounds of this invention are active as antibacterial agents. Their in vitro antibacterial activity was determined against a spectrum of gram-positive and gram-negative bacteria by a standard agar dilution method. Mueller-Hinton agar containing two-fold decreasing concentrations of the antibiotics was poured into petri dishes. The agar surfaces were inoculated with 1 to 5 x $10^4$ colony forming units of bacteria by means of a Steers replicating device. The lowest concentration of compound that inhibited growth of a bacterial strain after about 18 hours of incubation at approximately 36 °C. was recorded as the minimal inhibitory concentration (MIC) for that strain. The results are summarized in Table II.

Table II

*In vitro* Antibacterial Activity

| Organism | | Minimal Inhibitory Concentration (mcg/ml) LL-E33288$\gamma_2$-I | p-nitro phenyl ester of LL-E33288$\gamma_2$-I |
|---|---|---|---|
| *Escherichia coli* | CMC 84-11 | 0.25 | 0.5 |
| *Escherichia coli* | No. 311 | 0.25 | 0.5 |
| *Escherichia coli* | ATCC 25922 | 0.12 | 0.5 |
| *Klebsiella pneumoniae* | CMC 84-5 | 0.25 | 1 |
| *Klebsiella pneumoniae* | AD (MP) | 0.12 | 0.25 |
| *Enterobacter cloacae* | CMC 84-4 | 0.5 | 2 |
| *Enterobacter aerogenes* | IO 83-44 | 0.5 | 1 |
| *Serratia marcescens* | CMC 83-27 | 0.25 | 1 |
| *Serratia marcescens* | F-35 (MP) | 0.25 | 1 |
| *Morganella morganii* | IO 83-18 | 0.5 | 2 |
| *Providencia stuartii* | CMC 83-82 | 1 | 2 |
| *Citrobacter diversus* | K 82-24 | 0.5 | 1 |
| *Citrobacter freundii* | IO 83-13 | 0.5 | 0.5 |
| *Acinetobacter* sp. | CMC 83-89 | 0.25 | 0.5 |
| *Acinetobacter* sp. | IO 83-49 | 0.25 | 1 |
| *Pseudomonas aeruginosa* | 12-4-4 (MP) | 0.25 | 1 |
| *Pseudomonas aeruginosa* | ATCC 27853 | 0.12 | 0.5 |
| *Staphylococcus aureus* | Smith | 0.001 | 0.002 |
| *Staphylococcus aureus* | SSC 82-21 | 0.0005 | 0.004 |
| *Staphylococcus aureus* | ATCC 25923 | 0.0005 | 0.004 |
| *Staphylococcus aureus* | ATCC 29213 | 0.001 | 0.004 |
| *Staphylococcus aureus* | SSC 82-23 | 0.001 | 0.002 |
| *Staphylococcus aureus* | VGH 84-41 | 0.001 | 0.002 |
| *Staphylococcus aureus* | VGH 84-47 | 0.001 | 0.004 |

Table II (continued)

In vitro Antibacterial Activity

| Organism | | Minimal Inhibitory Concentration(mcg/ml) | |
|---|---|---|---|
| | | LL-E33288$\gamma_2$-I | p-nitro phenyl ester of LL-E33288$\gamma_2$-I |
| *Staphylococcus epidermidis* | CMC 83-133 | 0.001 | 0.002 |
| *Staphylococcus epidermidis* | ATCC 12228 | 0.001 | 0.004 |
| *Streptococcus faecalis* | ATCC 29212 | 0.002 | 0.004 |
| *Streptococcus faecalis* | VGH 84-65 | 0.004 | 0.004 |
| *Streptococcus faecalis* | CMC 83-53 | 0.004 | 0.004 |
| *Streptococcus faecalis* | UCI 85-20 | 0.002 | 0.004 |
| *Streptococcus faecalis* | IO 83-28 | 0.004 | 0.004 |

The disulfide analogs described above are active antitumor agents.

Certain in vivo testing systems and protocols have been developed by the National Cancer Institute for testing compounds to determine their suitability as antineoplastic agents. These have been reported in "Cancer Chemotherapy Reports", Part III, Vol. 3, No. 2 (1972), Geran, et al. These protocols have established standardized screening tests which are generally followed in the field of testing for antitumor agents. Of these systems, lymphocytic leukemia P388, melanotic melanoma B16, L1210 leukemia and colon 26 adenocarcinoma are particularly significant to the present invention. These neoplasms are utilized for testing as transplantable tumors in mice. Generally, significant antitumor activity, shown in these protocols by a percentage increase of mean survival times of the treated animals (T) over the control animals (C) is indicative of similar results in human leukemias and solid tumors.

Lymphocytic Leukemia P388 Test

The animals used were $BDF_1$ female mice. There were 5 or 6 mice per test group. The tumor transplant was by intraperitoneal injection of 0.5 ml of dilute ascitic fluid containing 1 x $10^6$ cells of lymphocytic leukemia P388 on day zero. The test compounds were administered intraperitoneally at a volume of 0.5 ml in sterile, pyrogen free saline on days 1, 5 and 9 (relative to tumor inoculation) at the indicated doses. The mice were weighed and survivors recorded on a regular basis for 30 days. The median survival time and the ratio of survival time for treated (T)/control (C) animals were calculated. The results appear in Table III.

Table III

| Lymphocytic Leukemia P388 Test | | | |
|---|---|---|---|
| Compound | Dose (mg/kg) | Median Survival (Days) | T/Cx100 % |
| LL-E33288$\gamma_2$-I (Methyldisulfide analog of LL-E33288$\gamma_1$-I) | 0.02 | 19.5 | 177 |
| | 0.0125 | 21 | 191 |
| | 0.01 | 19 | 173 |
| | 0.0063 | 26 | 236 |
| | 0.005 | 21 | 191 |
| | 0.003 | 20.5 | 178 |
| | 0.0025 | 18.5 | 168 |
| | 0.0015 | 17 | 154 |
| | 0.00125 | 15.5 | 141 |
| Control | - | 11.0 | - |
| Propyldisulfide analog of LL-E33288$\gamma_1$-I | 0.01 | 27.5 | *250* |
| | 0.005 | 21.5 | 195 |
| | 0.0025 | 16 | 145 |
| | 0.00125 | 14.5 | 131 |
| Control | - | 11.0 | - |

Melanotic Melanoma B16 Test

The animals used were BDF1 female mice. There were 5 or 6 mice per test group. A one gram portion of melanotic melanoma B16 tumor was homogenized in 10 ml of cold balanced salt solution and a 0.5 ml aliquot of the homogenate was implanted intraperitoneally into each of the test mice. The test compounds were administered intraperitoneally on days 1 through 9 (relative to tumor inoculation) at the indicated doses. The mice were weighed and survivors recorded on a regular basis for 60 days. The median survival time and the ratio of survival time for treated (T)/control (C) animals were calculated. The results appear in Table IV.

TABLE IV

| Melanotic Melanoma B16 Test | | | |
|---|---|---|---|
| Compound | Dose (mg/kg) | Median Survival (Days) | T/C x 100 (%) |
| LL-E33288$\gamma_2$-I (methyldisulfide analog of LL-E33288$\gamma_1$-I) | 0.005 | 34 | 162 |
| | 0.0025 | 38 | 181 |
| | 0.00125 | 33.5 | 160 |
| | 0.0006 | 28.5 | 136 |
| | 0.0003 | 26 | 124 |
| Control | - | 21 | - |

The invention will be further described in conjunction with the following examples.

Example 1

Methyldisulfide Analog of LL-E33288$\gamma_1$-I (LL-E33288$\gamma_2$-I)

A thiol reagent was prepared by bubbling methyl mercaptan gas into 10 ml of acetonitrile until it was essentially saturated. A 108 mg portion of LL-E33288$\gamma_1$-I was added to the thiol reagent, the resulting

suspension was then vortexed to produce a solution which was placed in a -20°C freezer overnight and then the solvent was evaporated. The residue was taken up in a small volume of ethyl acetate and this solution was dripped into 25 ml of stirred hexane giving an off-white precipitate that was dried, giving 82 mg of partially purified LL-33288$\gamma_2$-I.

The above 82 mg was treated with 0.1 ml of methanol and then 1 ml of methylene chloride was added. This solution was passed over 18 g of silica gel in a small column. The eluant used was 5% methanol in methylene chloride. The fractions were monitored by thin layer chromatography on silica gel using the system 10% isopropanol in ethyl acetate saturated with phosphate buffer. Fractions 4-11, containing LL-E33288$\gamma_2$-I were combined and evaporated. The residue was taken up in 0.5 ml of ethyl acetate and dripped into 20 ml of stirred hexane. The precipitate was collected and dried, giving 37 mg of LL-E33288$\gamma_2$-I, having ultraviolet spectra as shown in Figure I, a proton magnetic resonance spectrum as shown in Figure II, an infrared spectrum as shown in Figure III, and a mass spectrum as shown in Figure IV.

Example 2

Ethyldisulfide Analog of LL-E33288$\gamma$-I

The reaction described in Example 1 was repeated using a thiol reagent composed of ethyl mercaptan in acetonitrile, giving the desired ethyl disulfide analog of LL-E33288$\gamma_1$-I.

Example 3

Propyldisulfide Analog of LL-E33288$\gamma_1$-I

A 60 mg portion of LL-E33288$\gamma_1$-I was dissolved in 6 ml of acetonitrile and 5 ml of a 1 µmole/ml solution of propyl mercaptan in acetonitrile was added. After 3 hours, the reaction was essentially complete and the solvent was evaporated. The residue was taken up on 3 ml of ethyl acetate, filtered and the filtrate dripped into 40 ml of stirred hexane. The precipitate was collected and dried, giving 39 mg of the propyldisulfide analog of LL-E33288$\gamma_1$-I, having a proton magnetic resonance spectrum as shown in Figure V and a mass spectrum as shown in Figure VI.

Example 4

1-Methyl-1-propyl disulfide Analog of LL-E33288$\gamma_1$-I

A solution of 150 µg of LL-E33288$\gamma_1$-I in 1 ml of acetonitrile was treated with 50 µl of a 1 µmole/ml solution of 1-methyl-1-propyl mercaptan. After 2 hours, the reaction was complete, giving the desired 1-methyl-1-propyl disulfide analog of LL-E33288$\gamma_1$-I.

Example 5

t-Butyl disulfide Analog of LL-E33288$\gamma_1$-I

A 150 µg portion of LL-E33288$\gamma_1$-I in 1 ml of acetonitrile was treated with 50 µl of a 1µ mole/ml solution of t-butyl mercaptan in acetonitrile. After 2 1/4 hours, the reaction was complete, giving the t-butyl disulfide analog of LL-E33288$\gamma_1$-I.

Example 6

t-Butoxycarbonyl t-butyl cysteinyl disulfide Analog off LL-E33288$\gamma_1$-I

t-Butoxycarbonyl-cysteine-t-butyl ester was dissolved in acetonitrile at a concentration of 10 mg/ml. A 230 µg portion of LL-33288$\gamma_1$-I in acetonitrile was treated with 100 µl of the amino acid reagent. After 1 hour, the reaction was complete, giving the desired disulfide analog of LL-E33288$\gamma_1$-I.

Example 7

3-Mercaptopropionic acid disulfide Analog of LL-E33288$\gamma_1$-I

To a solution of 90 mg of LL-E33288$\gamma_1$-I in 90 ml of acetonitrile was added 10.6 mg of 3-mercaptopropionic acid in 1 ml of acetonitrile. The solution was vortexed and then stored at -20°C for 6 days. The solvent was removed in vacuo and the residue chromatographed over 10 ml of silica gel in methylene chloride. The column was developed with 50 ml of methylene chloride, 50 ml of 4% methanol in methylene chloride and finally 100 ml of 8% methanol in methylene chloride. Evaporation of this last fraction gave a residue which was taken up in ethyl acetate with the aid of a little acetone and added dropwise to an excess of hexane. The precipitate was collected and dried, giving 39 mg of the desired product (FABMS, M+H 1394).

Example 8

**Reaction of LL-E33288$\gamma_1$-I with the p-nitrophenyl ester of 3-mercaptopropionic acid**

**(A) Preparation of p-nitrophenyl ester of 3-mercaptopropionic acid**

Commercial 3-mercaptopropionic acid in methylene chloride containing a catalytic amount of concentrated sulfuric acid was treated with isobutylene for 20 minutes. The solution was then extracted with 1N sodium bicarbonate solution after which the methylene chloride solution was dried using anhydrous magnesium sulfate. The solution was then evaporated to a colorless mobile liquid which NMR and mass spectral data indicated was the 3-t-butyl mercaptopropionic acid, t-butyl ester.

An aliquot of this ester was refluxed with 6N hydrochloric acid in dioxane for 2.5 hours. The solvent was evaporated, ethyl acetate was added and this solution was extracted with sodium carbonate. The sodium carbonate extract was treated with 6N hydrochloric acid until the pH of the suspension was 2.0. The suspension was then extracted with ethyl acetate, the extract dried over anhydrous magnesium sulfate and the solvent evaporated to a colorless liquid which $^1$H NMR and mass spectral data indicated was 3-t-butyl mercaptopropionic acid.

This compound was converted to the p-nitrophenol ester by treatment with equimolar amounts of p-nitrophenol and dicyclohexylcarbodiimide in tetrahydrofuran for 4 hours. The dicyclohexyl urea by-product was removed by filtration and the filtrate was evaporated to an oil which was purified by passage over neutral silica gel using the solvent system hexane:methylene chloride (50:50). The pure p-nitrophenyl ester derivative was a faintly yellow, mobile oil.

The free mercaptan was unmasked by the following procedure. The 3-t-butyl mercaptopropionic acid p-nitrophenyl ester was dissolved in trifluoroacetic acid and a slight solar excess (10%) of mercuric acetate was added. The mixture was stirred for 30 minutes, then the trifluoroacetic acid was evaporated off and the residue taken up in dimethylformamide. This solution was treated with hydrogen sulfide gas for 15 minutes, then the black mercuric sulfide was filtered off and the filtrate evaporated under reduced pressure to eliminate up to 99% of the dimethylformamide. The resultant slightly brownish mobile liquid was purified over neutral silica gel using hexane:methylene chloride (50:50). The major component was shown by $^1$H NMR to contain a small amount of the t-butyl mercapto derivative. Analytical HPLC over two Perkin-Elmer Pecosphere $C_{18}$ columns in tandem [4.6 x 33 mm and 4.6 x 83 mm] using a gradient system of 37.5/62.5 to 47.5/52.5 of acetonitrile and 0.1M ammonium acetate buffer at pH 6.5 (acetic acid) over a 12 minute span indicated that the product was 88% of the p-nitrophenyl ester of 3-mercaptopropionic acid and 10% of the less polar 3-t-butyl mercaptopropionic acid p-nitrophenyl ester. There was also a small amount of free p-nitrophenol present.

**(B) Reaction of p-nitrophenyl ester of 3-mercaptopropionic acid with LL-E33288$\gamma_1$-I**

A 100 mg portion of LL-E33288$\gamma_1$-I was dissolved in 50 ml of acetonitrile. To this was added a solution of 25.7 mg of p-nitrophenyl ester of 3-mercaptopropionic acid in 1 ml of acetonitrile. The reaction was left at -20°C for 48 hours. HPLC indicated the reaction was complete. The solution was evaporated to dryness and the residue taken up in 4-5 ml of ethyl acetate using sonication to effect solution. The mixture was filtered and the filtrate dripped into 45 ml of stirred hexane. The resultant faintly yellow solid was collected and dried under reduced pressure, giving 93 mg of the p-nitrophenyl ester of propionic acid derivative of LL-E33288$\gamma_2$-I as established by $^1$H NMR. By FABMS the [M+H] ion appeared at M/Z = 1515.

## Example 9

### 7-(2-Mercaptoethylamino)-4-methyl coumarin disulfide Analog of LL-E33288$\gamma_1$-I

To 10 mg of 70% pure LL-E33288$\gamma_1$-I in 9 mL of acetonitrile was added 1.2 mg of 7-(2-thioethylamino)-4-methyl coumarin in 1 mL acetonitrile. After stirring for 36 hours at 0°C the solvent was stripped and the product was chromatographed on silica gel with 5% methanol in chloroform to give 5 mg of the desired product. Retention time on a C-18 reverse phase HPLC column: 5.4 min with 56% acetonitrile, 10.1M aqueous ammonium chloride. (LL-E33288$\delta_1$-I retention time in the same system is 5.5 min.).

## Example 10

### 3-Mercaptopropionyl hydrazide disulfide analog of LL-E33288$\Psi_1^I$

To 5.4 ml (3 eq) of anhydrous hydrazine in 100 ml of refluxing tetrahydrofuran under argon was added dropwise 9.2 ml (83 mmol) of methyl 3-mercaptopropionate in 50 ml tetrahydrofuran over 2 hours. The solution was refluxed an additional two hours, evaporated, and then diluted and evaporated twice from 300 ml of toluene. The product was applied to a plug of silica gel with 5% ethyl acetate/chloroform and eluted from the plug with 20% methanol/chloroform. The resultant 3-mercaptopropionyl hydrazide was a faintly pink oil which solidified when cooled but melted at room temperature.

To 50 mg of LL-E33288$\Psi_1^I$ in 50 ml of acetonitrile at -15°C was added 6.6 mg of 3-mercaptopropionyl hydrazide in 1 ml tetrahydrofuran. One equivalent of triethylamine and/or 1 equivalent of acetic acid was added as catalyst. The reaction was allowed to stir at 0°C for 1 hour and the solvent was then evaporated. The residue was chromatographed on silica gel with a 10-15% methanol in chloroform gradient to yield 26 mg of the desired product. Retention time on reverse phase $C_{18}$ HPLC:5.0 min in 41% acetonitrile/0.1M aqueous ammonium chloride.

## Example 11

### N-[[(4-Methyl-coumarin-7-yl)amino]acetyl]cysteine hydrazide disulfide analog of LL-E33288$\Psi_1^I$

A mixture of 1.0g (5.7 mmol) of 4-methyl-7-aminocourmarin, 3.0 ml of ethyl bromoacetate (5 eq), 90 mg (0.1 eq) of sodium iodide, and 30 ml dimethylformamide was heated under argon at 80°C for 5 hours. The mixture was cooled, diluted with ethyl ether, washed three times with 50% brine, dried with magnesium sulfate, and evaporated to dryness. The crude product was dissolved in chloroform containing 1% ethyl acetate and filtered through a plug of silica gel. Recrystallization from diethyl ether containing a trace of chloroform yielded pure ethyl N-[(4-methylcourmarin-7-yl)amino]acetate.

To 1.96 g (7.5 mmol) of the above ester in 15 ml of methanol and 15 ml of tetrahydrofuran was added 10 ml of 1N aqueous sodium hydroxide. After 30 minutes, 4 ml of 10% aqueous hydrochloric acid was added. The organic solvents were evaporated and the resultant crystalline product was filtered and washed with cold ethanol and then ether. This material was dissolved in 20 ml of tetrahydrofuran and 4 ml of dimethylformamide. Dicyclohexylcarbonyldiimidazole (1.3 g, 2.2 eq) was added and the reaction allowed to stir for 15 minutes. Cysteine ethyl ester hydrochloride (1.6 g, 2.5 eq) and triethylamine (1.2 ml) were then added. After a further three hours, the reaction was diluted with ethyl ether containing 5% methylene chloride and washed once with 10% aqueous hydrochloric acid and twice with brine. After drying with magnesium sulfate and evaporating the solvents, the crude product was crystallized by dissolving in chloroform containing a minimal amount of ethanol and then adding an excess of ether. The crystals were filtered and dried to give pure N-[[(4-methyl-coumarin-7-yl)amino]acetyl]cysteine ethyl ester.

A mixture of 5 ml of chloroform, 20 ml of methanol, and 0.4 ml of hydrazine hydrate were heated to reflux under argon. To this was added 550 mg of N-[[(4-methyl-coumarin-7-yl)amino]acetyl]cysteine ethyl

ester. After refluxing for 9 hours the mixture was cooled and the solid product was filtered and washed with chloroform and then ethyl ether. The crude product (which contained thiol and disulfide) was dissolved in dimethylformamide containing dithiothreitol and triethyl amine. After 30 minutes the product was precipitated with excess ethyl ether and collected by filtration. This material was purified further by recrystallization from degassed acetonitrile containing dithiothreitol and a trace of triethyl amine to give pure N-[[(4-methyl-coumarin-7-yl)amino]acetyl]cysteine hydrazide.

To 12 mg of 70% pure LL-E33288$\gamma_1^I$ in 12 ml acetonitrile at 0°C was added 4 mg of N-[[(4-methyl-coumarin-7-yl)amino]acetyl]cysteine hydrazide in 1.2 ml dimethylformamide. After stirring overnight another 2 mg of N-[[(4-methyl-coumarin-7-yl)amino]cysteine hydrazide in 0.6 ml dimethylformamide was added. The reaction was stirred for 3 days at 0°C and filtered. The acetonitrile was evaporated and the resultant dimethylformamide solution was diluted with an excess of 1:1 hexanes/ether. The product was isolated by filtration and further purified by chromatography on silica gel with a 15-20% gradient of methanol in chloroform to yield 3 mg of the desired product. Retention time on reverse phase $C_{18}$ HPLC: 3.5 minutes using 45% acetonitrile/0.1 M aqueous ammonium chloride.

Example 12

3-Mercaptopropionyl hydrazide disulfide analog of LL-E33288$\alpha_3^I$

To 10 mg of LL-E33288$\alpha_3^I$ in mL of acetonitrile at -15°C was added 6.6 mg of 3-mercaptopropionyl hydrazide in 1 mL acetonitrile. One equivalent of triethylamine and/or 1 equivalent of acetic acid were added as a catalyst. The reaction was allowed to stir at 0°C for 1 hour and the solvent was then evaporated. The residue was chromatographed on silica gel with a 10-15% methanol in chloroform gradient to give the desired product. Retention time on reverse phase $C_{18}$ HPLC:3.5 min. in the system 45% acetonitrile 10.1M aqueous ammonium chloride.

## Claims

**Claims for the following Contracting States : AT, BE, CH, DE, FR, GB, IT, LI, NL, SE**

**1.** The antitumor agent LL-E33288$\gamma_2$-I having

a) ultraviolet spectra as shown in Figure I;
b) a proton magnetic resonance spectrum as shown in Figure II;
c) an infrared spectrum as shown in Figure III;
d) a mass spectrum as shown in Figure IV and
e) the following formula

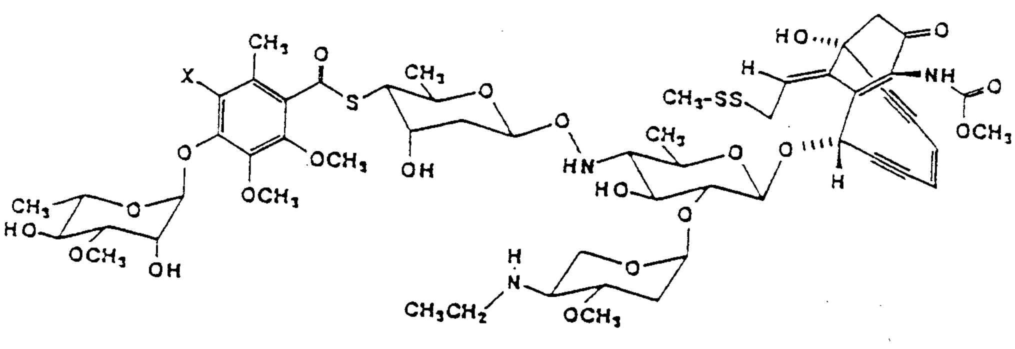

R = $CH_3$, X = I = LL33288$\gamma_2^I$

**2.** The antitumor agent LL-E33288$\gamma_2$-Br having the following formula

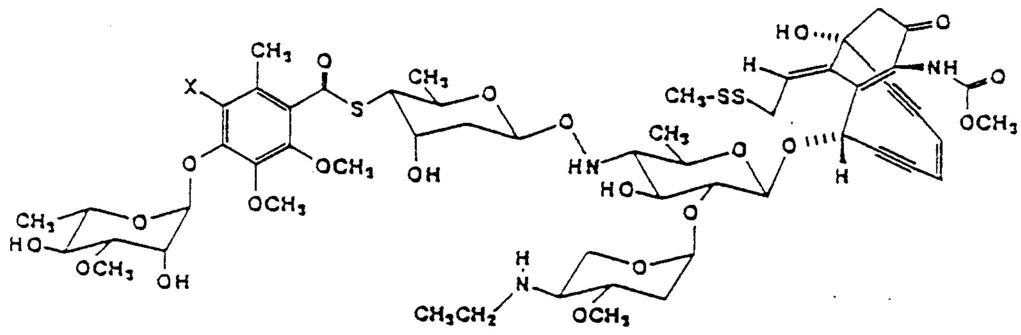

R = $CH_3$, X = Br = LL33288$\gamma_2^{Br}$

**3.** A substituted disulfide of the formula Q-Sp-SS-W, prepared from a compound of the formula $CH_3SSS$-W and designated as antibiotic LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$I, $\delta_1$-I, BBm-1675, FR-900405, FR-900406, PD 114759, PD 115028, CD-1577A, CL-1577B, CL-1577D, CL-1571E, or CL-1724 wherein

W is

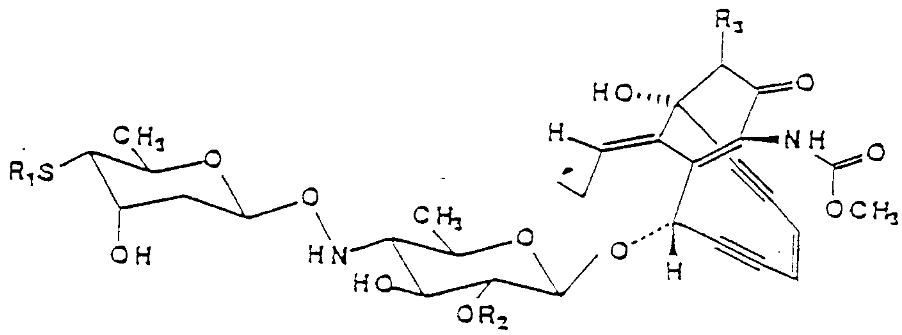

$R_1$ is

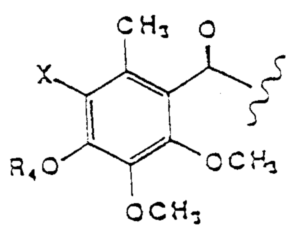

or $CH_3$,

$R_2$ is

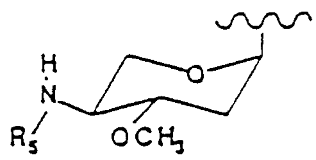

or H,

$R_3$ is

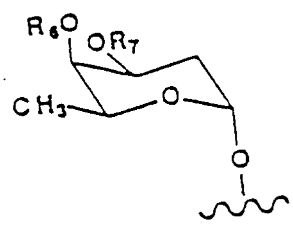

or H,

$R_4$ is

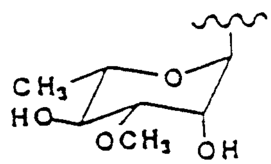

or H,

$R_5$ is $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ or
$CH_3CH_2$,

$R_6$ is

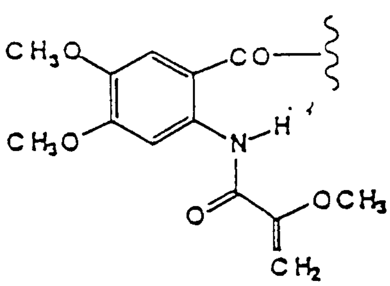

or H,

$R_7$ is

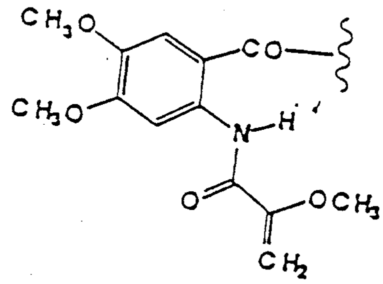

or H

and X is iodine or bromine; Sp is straight or branched-chain divalent($C_1$-$C_{18}$) radicals; divalent aryl or heteroaryl radicals, divalent ($C_3$-$C_{18}$)cycloalkyl radicals, divalent($C_1$-$C_{18}$) aryl- or heteroaryl-alkyl radicals, divalent($C_1$-$C_{18}$) cycloalkyl-alkyl radicals, or divalent($C_2$-$C_{18}$) unsaturated alkyl radicals; Q is hydrogen, halogen, amino, alkylamino, dialkylamino, piperidino, pyrrolidino, piperazino, carboxyl, carboxaldehyde, lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the proviso that when Sp is ethylidine, Q cannot be hydrogen.

**4.** The ethyl disulfide analog of LL-E33288$\gamma_1$-I.

**5.** The propyl disulfide analog of LL-E33288$\gamma_1$-I having a proton magnetic resonance spectrum as shown in Figure V and a mass spectrum as shown in Figure VI.

**6.** The 1-methyl-1-propyl disulfide analog of LL-E33288$\gamma_1$-I,

**7.** A compound according to claim 3 wherein the heteroarylalkyl radical is 7-amino-4-methyl-coumarin.

**8.** A process for producing the substituted disulfide of the formula Q-Sp-SS-W, prepared from a compound of the formula $CH_3SSS$-W and designated as antibiotic LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E, or CL-1724 wherein

W is

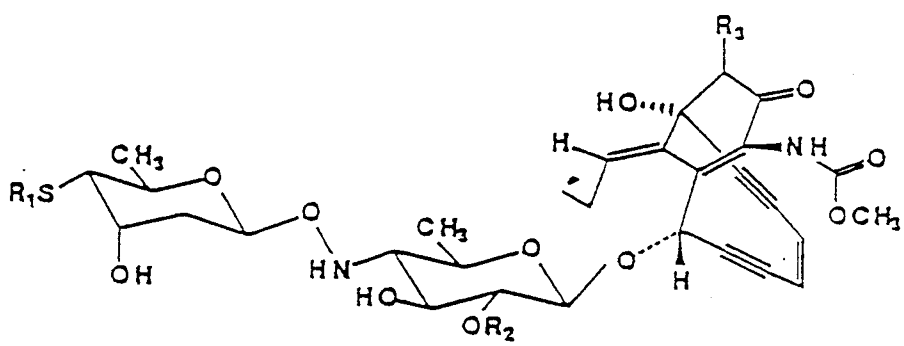

$R_1$ is

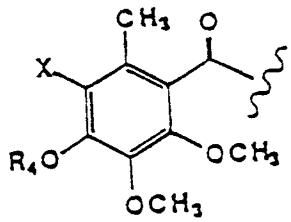

or $CH_3$,

$R_2$ is

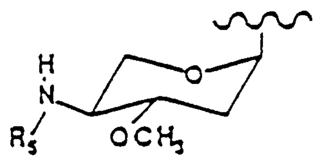

or H,

$R_3$ is

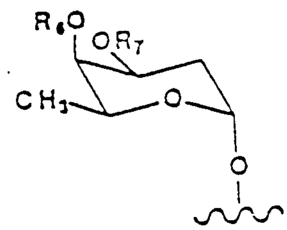

or H,

$R_4$ is

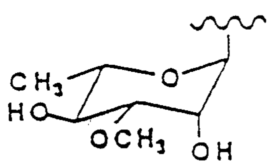

or H,

$R_5$ is $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ or
$CH_3CH_2$,

$R_6$ is

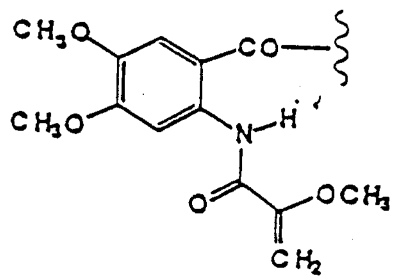

or H,

$R_7$ is

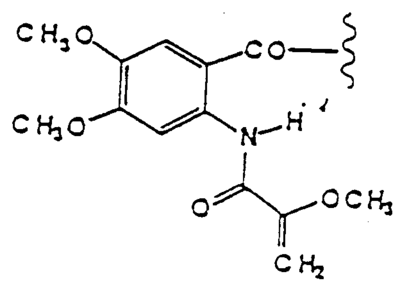

or H

and X in iodine or bromine; Sp is straight or branched-chain divalent($C_1$-$C_{18}$) radicals; divalent aryl or heteroaryl radicals, divalent ($C_3$-$C_{18}$) cycloalkyl radicals, divalent ($C_1$-$C_{18}$) aryl- or heteroaryl-alkyl radicals, divalent($C_1$-$C_{18}$) cycloalkyl-alkyl radicals, or divalent($C_2$-$C_{18}$) unsaturated alkyl radicals; Q is hydrogen, halogen, amino, alkylamino, dialkylamino, piperidino, pyrrolidino, piperazino, carboxyl, carboxaldehyde, lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the proviso that when Sp is ethylidine, Q cannot be hydrogen; which comprises reacting an alkyl trisulfide antibiotic from those of the above with an appropriated substituted mercaptan in acetonitrile at -10° to -30°C for 1-48 hours.

**9.** The use of a substituted disulfide of the formula Q-Sp-SS-W, prepared from a compound of the formula $CH_3SSS$-W and designated as antibiotic LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-BR, $\gamma_1$I, $\delta_1$-I, BBm-1675, FR-900405, FR-900406, PD 114759, PD 115028, CL-1577A, CL-1577B, CL-1577D, CL-1577E, wherein

W is

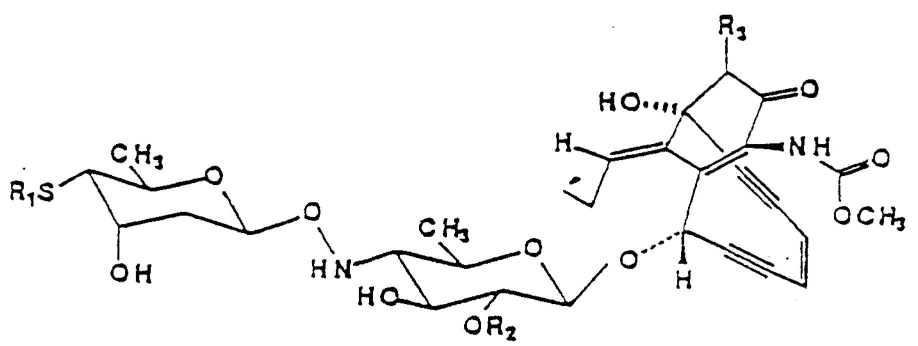

$R_1$ is

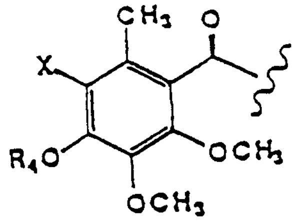

or $CH_3$,

$R_2$ is

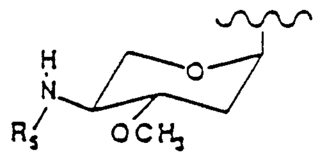

or H,

$R_3$ is

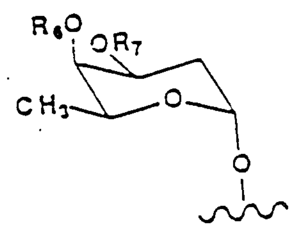

or H,

$R_4$ is

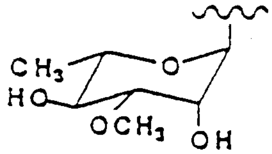

or H,

$R_5$ is $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ or

$CH_3CH_2$,

$R_6$ is

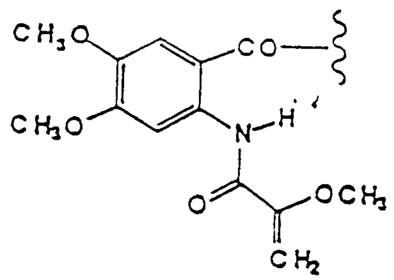

or H,

$R_7$ is

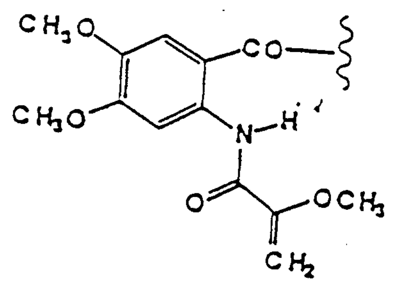

or H

and X is iodine or bromine; Sp is straight or branched-chain divalent($C_1$-$C_{18}$) radicals; divalent aryl or heteroaryl radicals, divalent ($C_3$-$C_{18}$)cycloalxyl radicals, divalent($C_1$-$C_{18}$) aryl- heteroaryl-alkyl radicals, divalent($C_1$-$C_{18}$) cycloalky-lalkyl radicals, or divalent($C_2$-$C_{18}$) unsaturated alkyl radicals; Q is hydrogen, halogen, amino, alkylamino, dialkylamino, piperidino, pyrrolidino, piperazino, carboxyl, carboxaldehyde; lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the proviso that when Sp is ethylidine, Q cannot be hydrogen for the manufacture of a medicament for treating bacterial infections in warm-blooded animals.

**10.** The use of a substituted disulfide of the formula Q-Sp-SS-W, prepared from a compound of the formula $CH_3SSS$-W and designated as antibiotic LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$I, $\delta_1$-I, BBm-1675, FR-900405, FR-900406, PD 114759, PD 115028, CL-1577A, CL-1577B, CL-1577D, CL-1577E, or CL-1724 wherein

W is

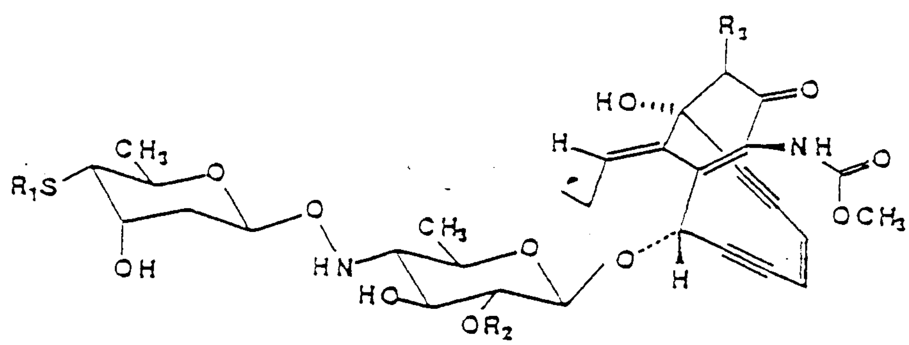

$R_1$ is

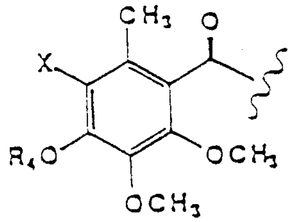

or $CH_3$,

$R_2$ is

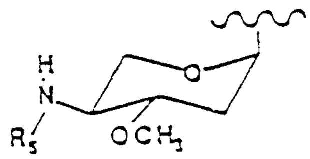

or H,

$R_3$ is

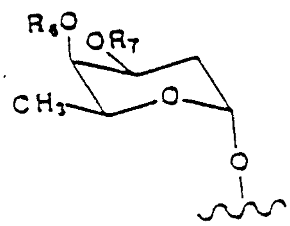

or H,

$R_4$ is

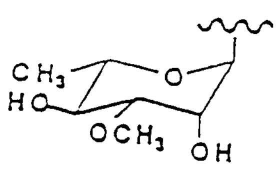

or H,

$R_5$ is $C_2H_5$
$(CH_3)_2CH$
$C_2H_5$
$CH_3$
$(CH_3)_2CH$
$C_2H_5$
$C_2H_5$
$(CH_3)_2CH$

(CH3)2CH or
CH3CH2,

R6 is

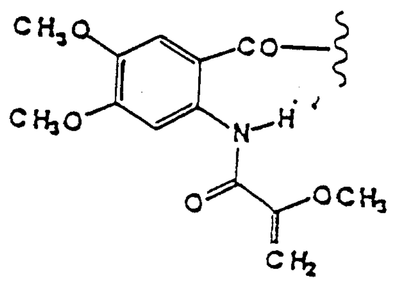

or H,

R7 is

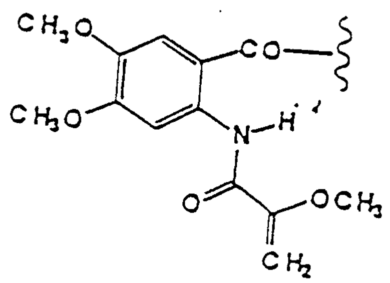

or H

and X is iodine or bromine; Sp is straight or branched-chain divalent($C_1$-$C_{18}$) radicals; divalent aryl or heteroaryl radicals, divalent ($C_3$-$C_{18}$)cycloalxyl radicals, divalent($C_1$-$C_{18}$) aryl- heteroaryl-alkyl radicals, divalent($C_1$-$C_{18}$) cycloalkyl-alkyl radicals, or divalent($C_2$-$C_{18}$) unsaturated alkyl radicals; Q is hydrogen, halogen, amino, alkylamino, dialkylamino, piperidino, pyrrolidino, piperazino, carboxyl, carboxaldehyde; lower alkoxy, hydroxy, thiol, lower alkyldicarboxyl, or a group chosen from the following: $-CONHNH_2$, $-NHCONHNH_2$, $-CSNHNH_2$, $-NHCSNHNH_2$, or $-ONH_2$; with the proviso that when Sp is ethylidine, Q cannot be hydrogen for the manufacture of a medicament for inhibiting the growth of tumors in a mammal.

**Claims for the following Contracting States : ES, GR**

**1.** A process for producing the substituted disulfide of the formula Q-Sp-SS-W, prepared from a compound of the formula $CH_3SSS$-W and designated as an antibiotic LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$I, $\delta_1$-I, BBm-1675, FR-900405, FR-900406, PD 114759, PD 115028, CL-1577A, CL-1577B, CL-1577D, CL-1577E, or CL-1724, wherein

W is

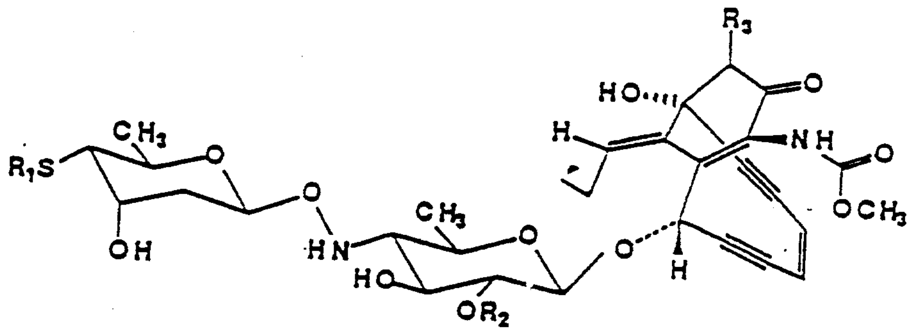

$R_1$ is

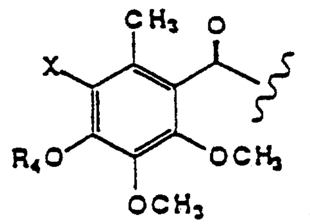

or $CH_3$,

$R_2$ is

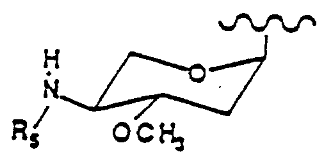

or H,

$R_3$ is

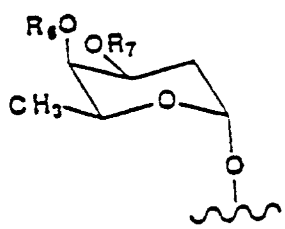

or H,

$R_4$ is

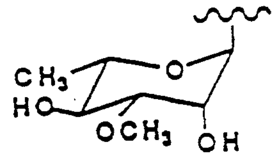

or H,

$R_5$ is $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ or
$CH_3CH_2$,

$R_6$ is

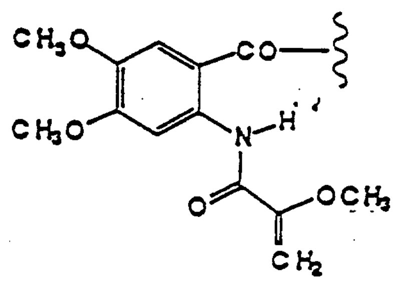

or H,

$R_7$ is

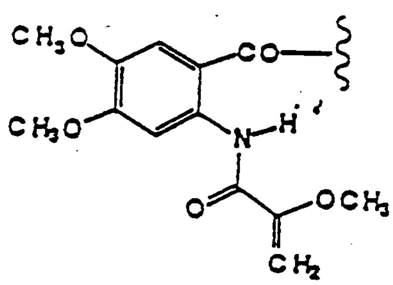

or H

and X is iodine or bromine; Sp is straight or branched-chain divalent ($C_1$-$C_{18}$) radicals; divalent aryl or heteroaryl radicals, divalent ($C_3$-$C_{18}$)cycloalkyl radicals, divalent ($C_1$-$C_{18}$) arylalkyl or heteroarylalkyl radicals, divalent($C_1$-$C_{18}$) cycloalkylalkyl radicals, or divalent($C_2$-$C_{18}$) unsaturated alkyl radicals: Q is hydrogen, halogen, amino, alkylamino, dialkylamino, piperidino, pyrrolidino, piperazino, carboxyl, carboxaldehyde, lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the proviso that when Sp is ethylidine, Q cannot be hydrogen: which comprises reacting an alkyl trisulfide antibiotic from those of the above with an appropriated substituted mercaptan in acetonitrile at -10 °C to -30 °C for 1-48 hours.

**2.** The process of Claim 1 which produces the antitumor agent LL-E33288$\gamma_2$-I having

a) ultraviolet spectra as shown in Figure I;
b) a proton magnetic resonance spectrum as shown in Figure II;
c) an infrared spectrum as shown in Figure III;
d) a mass spectrum as shown in Figure IV and
e) the following formula

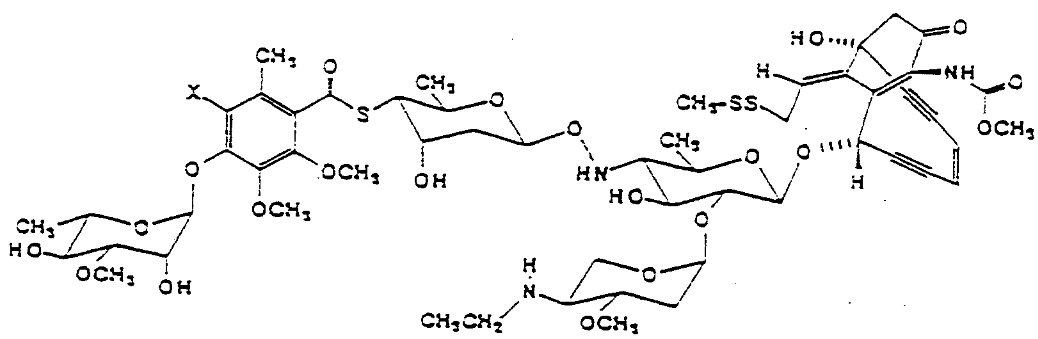

$R = CH_3$, $X = I$ = LL33288$\gamma_2^{I}$

**3.** The process according to Claim 1 which produces the antitumor agent LL-E33288$\gamma_2$-Br having the following formula

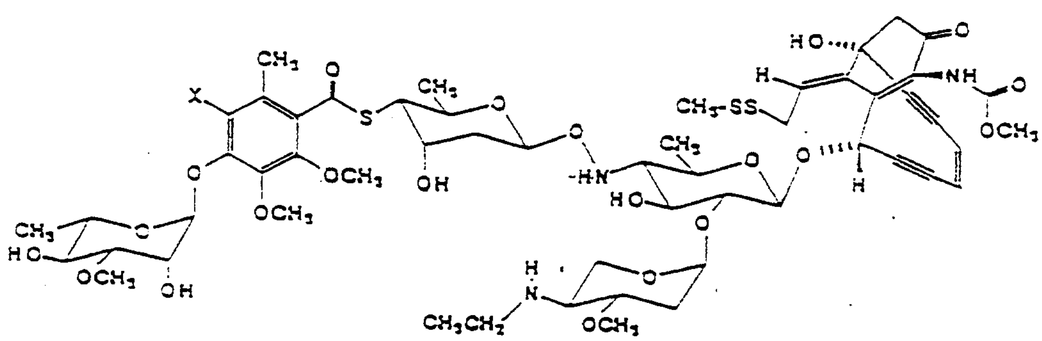

$R = CH_3$, $X = Br$ = LL33288$\gamma_2^{Br}$

**4.** The process according to Claim 1 which produces the ethyl disulfide analog of LL-E33288$\gamma_1$-I.

**5.** The process according to Claim 1 which produces the propyl disulfide analog of LL-E33288$\gamma_1$-I having a proton magnetic resonance spectrum as shown in Figure V and a mass spectrum as shown in Figure VI.

**6.** The process according to Claim 1 which produces the 1-methyl-1-propyl disulfide analog of LL-E33288$\gamma_1$-I.

**7.** The process of Claim 1, wherein the heteroarylalkyl radical is 7-amino-4-methyl-coumarin.

## Patentansprüche

**Patentansprüche für folgende Vertragsstaaten : AT, BE, CH, DE, FR, GB, IT, LI, NL, SE**

1. Antitumormittel LL-E33288$\gamma_2$-I mit
   a) einem UV-Spektrum, wie in Figur I gezeigt;
   b) einem protonenmagnetischen Resonanzspektrum, wie in Figur II gezeigt;
   c) einem IR-Spektrum, wie in Figur III gezeigt;
   d) einem Massenspektrum, wie in Figur IV gezeigt und
   e) der folgenden Formel

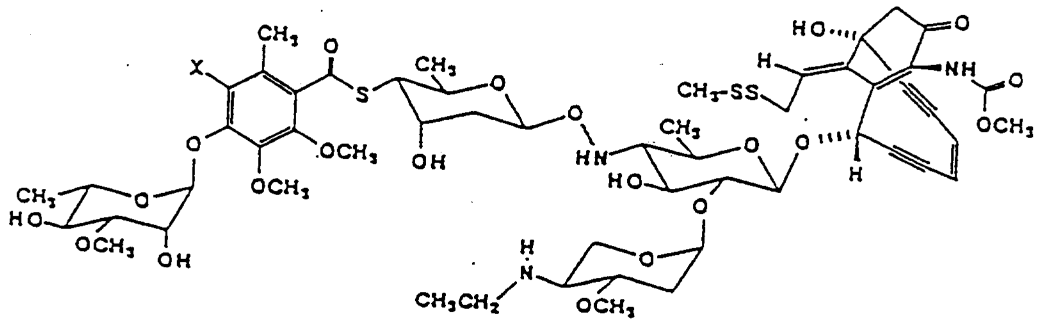

$$R = CH_3,\ X = I = \text{LL-E33288}\gamma_2{}^{I}.$$

2. Antitumormittel LL-E33288$\gamma_2$-Br der folgenden Formel

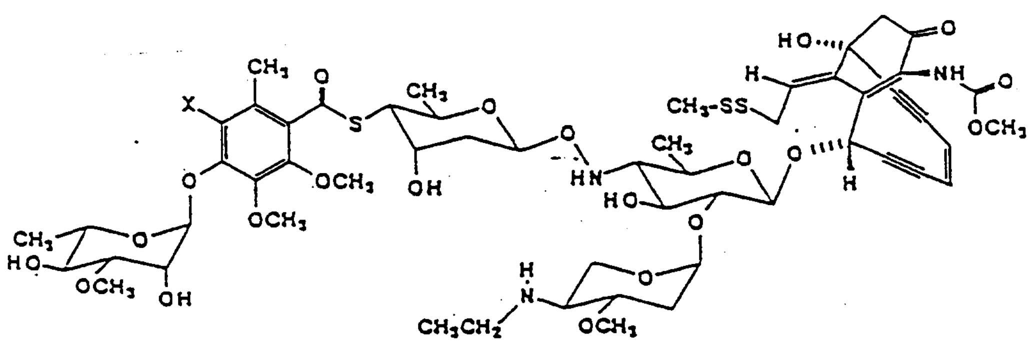

$$R = CH_3,\ X = Br = \text{LL-E33288}\gamma_2{}^{Br}.$$

3. Substituiertes Disulfid der Formel Q-Sp-SS-W, hergestellt aus einer Verbindung der Formel $CH_3SSS$-W und bezeichnet als Antibiotikum LL-E33288$\alpha_1$-Br, $\alpha_1$-I, $\alpha_2$-Br, $\alpha_2$-I, $\alpha_3$-Br, $\alpha$3-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E oder CL-1724, worin
   W ist

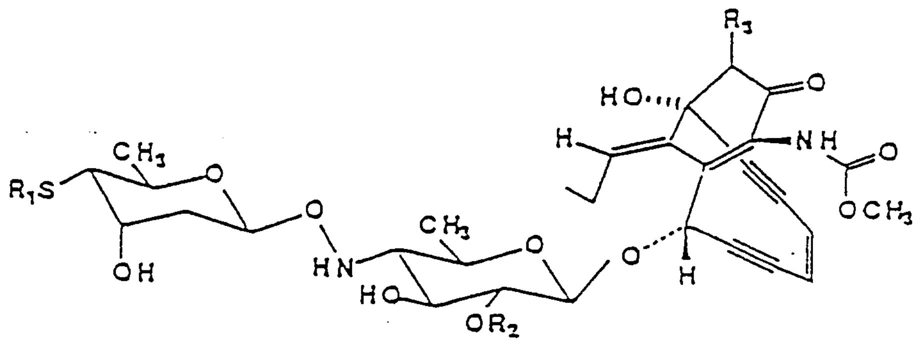

$R_1$ ist

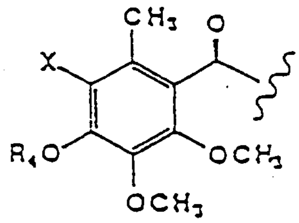

oder $CH_3$,

$R_2$ ist

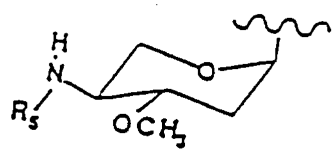

oder H,

$R_3$ ist

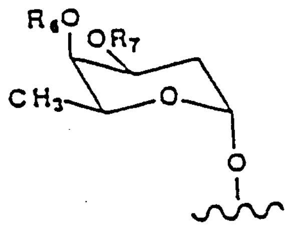

oder H,

$R_4$ ist

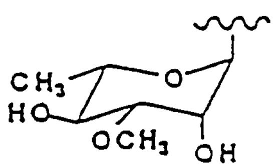

oder H,

$R_5$ ist $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ oder
$CH_3CH_2$

$R_6$ ist

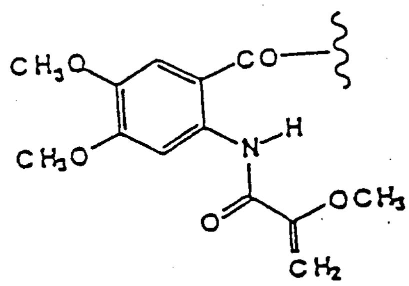

oder H,

$R_7$ ist

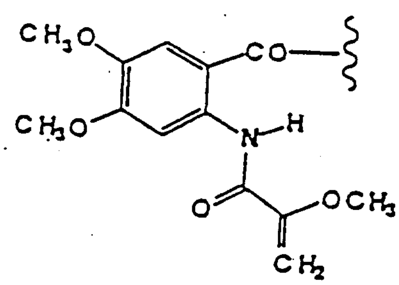

oder H

und X ist Jod oder Brom; Sp ist geradkettiger oder verzweigter zweiwertiger $C_1$-$C_{18}$-Rest; zweiwertiger Aryl- oder Heteroarylrest, zweiwertiger $C_3$-$C_{18}$-Cycloalkylrest, zweiwertiger $C_1$-$C_{18}$-Aryl- oder Heteroaryl-Alkylrest, zweiwertiger $C_1$-$C_{18}$-Cycloalkyl-alkylrest oder zweiwertiger ungesättigter $C_2$-$C_{18}$-Alkylrest; Q ist Wasserstoff, Halogen, Amino, Alkylamino, Dialkylamino, Piperidino, Pyrrolidino, Piperazino, Carboxyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$, unter der Bedingung, daß, wenn Sp Ethyliden ist, Q nicht Wasserstoff sein kann.

**4.** Das Ethyldisulfid-Analoge von LL-E33288$\gamma_1$-I.

**5.** Das Propyldisulfid-Analoge von LL-E33288$\gamma_1$-I mit einem Protonenmagnetischen Resonanzspektrum, wie in Figur V gezeigt und einem Massenspektrum, wie in Figur VI gezeigt.

**6.** Das 1-Methyl-1-propyldisulfid-Analoge von LL-E33288$\gamma_1$-I.

**7.** Verbindung nach Anspruch 3, worin der Heteroalkylrest 7-Amino-4-methyl-cumarin ist.

**8.** Verfahren zum Herstellen des substituierten Disulfids der Formel Q-Sp-SS-W, hergestellt aus einer Verbindung der Formel $CH_3SSS$-W und bezeichnet als Antibiotikum LL-E33288$\alpha_1$-Br, $\alpha_1$-I, $\alpha_2$-Br, $\alpha_2$-I,

$\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E oder CL-1724, worin

W ist

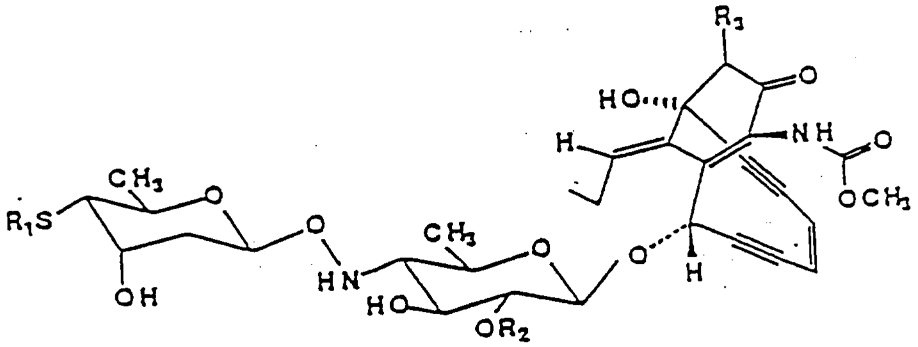

$R_1$ ist

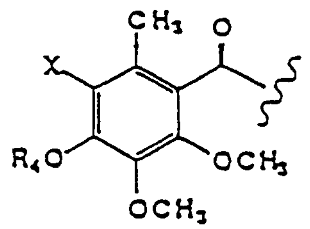

oder $CH_3$,

$R_2$ ist

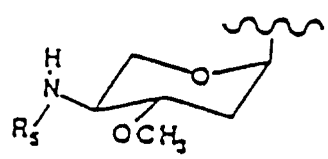

oder H,

$R_3$ ist

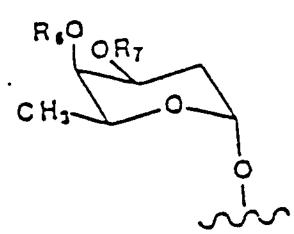

oder H,

$R_4$ ist

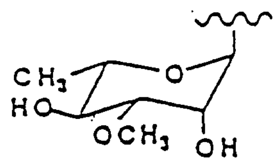

oder H,

$R_5$ ist $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ oder
$CH_3CH_2$

$R_6$ ist

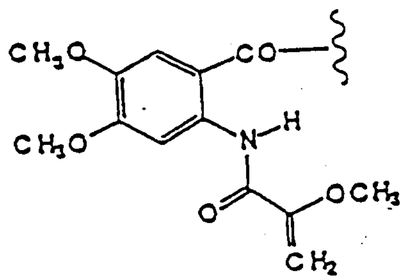

oder H,

$R_7$ ist

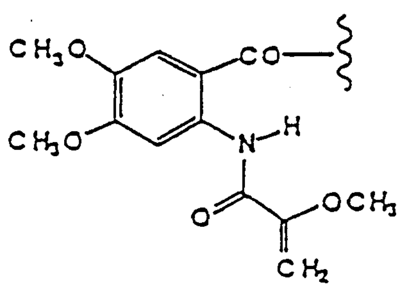

oder H

und X ist Jod oder Brom; Sp ist geradkettiger oder verzweigter zweiwertiger $C_1$-$C_{18}$-Rest; zweiwertiger Aryl- oder Heteroarylrest, zweiwertiger $C_3$-$C_{18}$-Cycloalkylrest, zweiwertiger $C_1$-$C_{18}$-Aryl- oder Heteroaryl-Alkylrest, zweiwertiger $C_1$-$C_{18}$-Cycloalkyl-alkylrest oder zweiwertiger ungesättigter $C_2$-$C_{18}$-Alkylrest; Q ist Wasserstoff, Halogen, Amino, Alkylamino, Dialkylamino, Piperidino, Pyrrolidino, Piperazino, Carboxyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$, unter der Bedingung, daß, wenn Sp Ethyliden ist, Q nicht Wasserstoff sein kann, umfassend die Umsetzung eines Alkyltrisulfid-Antibiotikums aus den obigen mit einem geeignet substituierten Mercaptan in Acetonitril bei -10 °C bis -30 °C für 1-48 Stunden.

**9.** Verwendung eines substituierten Disulfids der Formel Q-Sp-SS-W, hergestellt aus einer Verbindung der Formel $CH_3SSS$-W und bezeichnet als Antibiotikum LL-E33288$\alpha_1$-Br, $\alpha_1$-I, $\alpha_2$-Br, $\alpha_2$-I, $\alpha_3$-Br, $\alpha_3$-I, $\alpha_4$-

Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E, worin

W ist

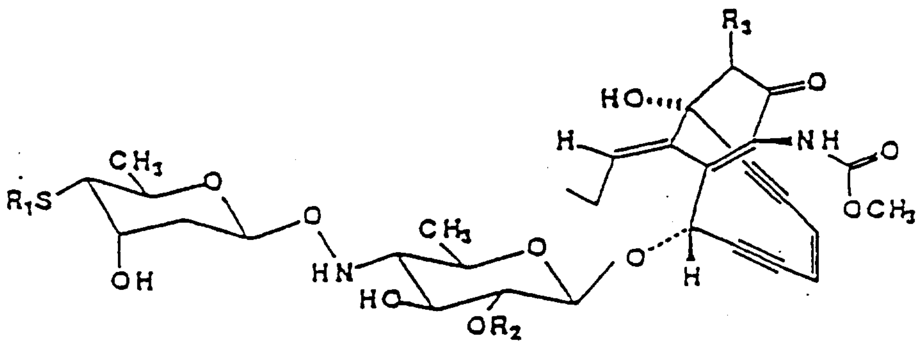

$R_1$ ist

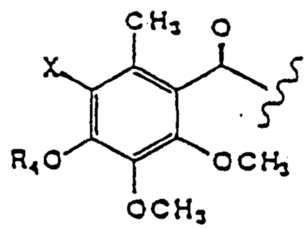

oder $CH_3$,

$R_2$ ist

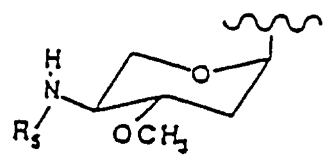

oder H,

$R_3$ ist

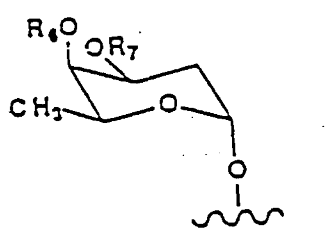

oder H,

$R_4$ ist

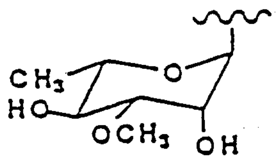

oder H,

$R_5$ ist $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ oder
$CH_3CH_2$

$R_6$ ist

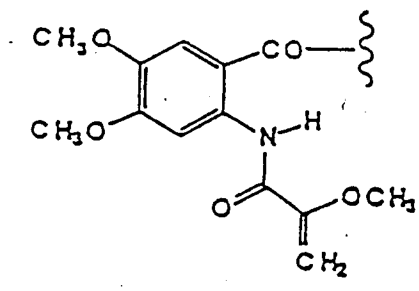

oder H,

$R_7$ ist

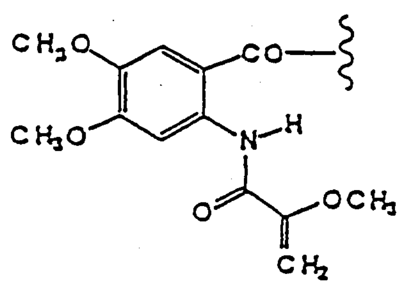

oder H

und X ist Jod oder Brom; Sp ist geradkettiger oder verzweigter zweiwertiger $C_1$-$C_{18}$-Rest; zweiwertiger Aryl- oder Heteroarylrest, zweiwertiger $C_3$-$C_{18}$-Cycloalkylrest, zweiwertiger $C_1$-$C_{18}$-Aryl- oder Heteroaryl-Alkylrest, zweiwertiger $C_1$-$C_{18}$-Cycloalkyl-alkylrest oder zweiwertiger ungesättigter $C_2$-$C_{18}$-Alkylrest; Q ist Wasserstoff, Halogen, Amino, Alkylamino, Dialkylamino, Piperidino, Pyrrolidino, Piperazino, Carboxyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$, unter der Bedingung, daß, wenn Sp Ethyliden ist, Q nicht Wasserstoff sein kann, für die Herstellung eines Medikaments zur Behandlung bakterieller Infektionen in warmblütigen Tieren.

10. Verwendung eines substituierten Disulfids der Formel Q-Sp-SS-W, hergestellt aus einer Verbindung der Formel $CH_3SSS$-W und bezeichnet als Antibiotikum LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E oder CL-1724, worin

W ist

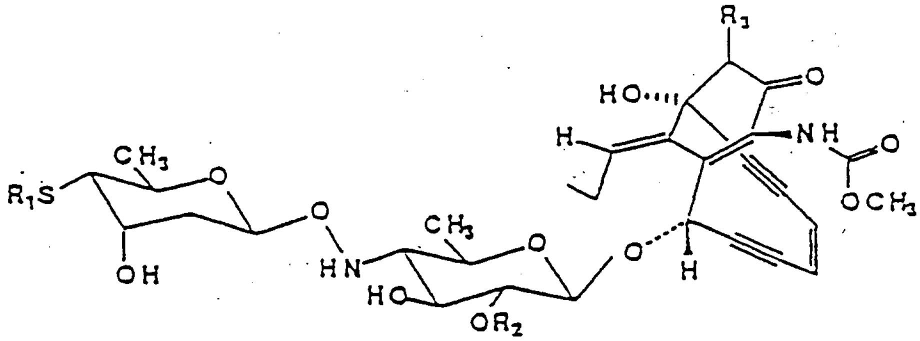

$R_1$ ist

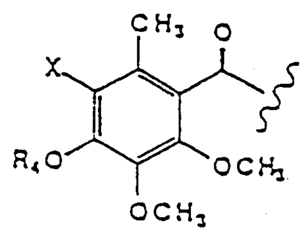

oder $CH_3$,

$R_2$ ist

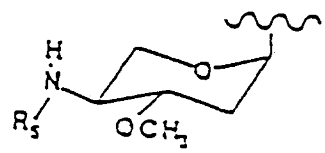

oder H,

$R_3$ ist

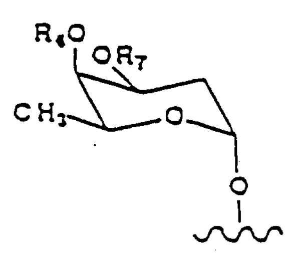

oder H,

$R_4$ ist

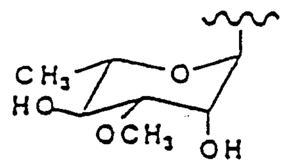

oder H,

$R_5$ ist $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ oder
$CH_3CH_2$

$R_6$ ist

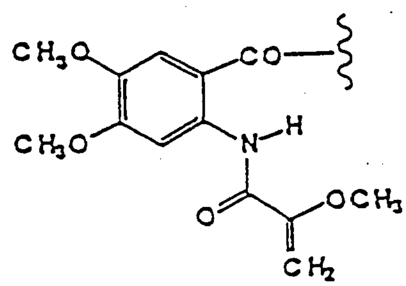

oder H,

$R_7$ ist

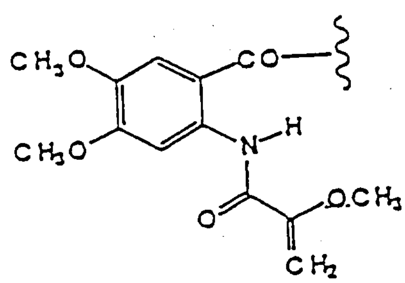

oder H

und X ist Jod oder Brom; Sp ist geradkettiger oder verzweigter zweiwertiger $C_1$-$C_{18}$-Rest; zweiwertiger Aryl- oder Heteroarylrest, zweiwertiger $C_3$-$C_{18}$-Cycloalkylrest, zweiwertiger $C_1$-$C_{18}$-Aryl- oder Heteroaryl-Alkylrest, zweiwertiger $C_1$-$c_{18}$-Cycloalkyl-alkylrest oder zweiwertiger ungesättigter $C_2$-$C_{18}$-Alkylrest; Q ist Wasserstoff, Halogen, Amino, Alkylamino, Dialkylamino, Piperidino, Pyrrolidino, Piperazino, Carboxyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$, unter der Bedingung, daß, wenn Sp Ethyliden ist, Q nicht Wasserstoff sein kann, zur Herstellung eines Medikaments zum Hemmen des Wachstums von Tumoren in einem Säugetier.

**Patentansprüche für folgende Vertragsstaaten : ES, GR**

1. Verfahren zum Herstellen des substituierten Disulfids der Formel Q-Sp-SS-W, hergestellt aus einer Verbindung der Formel $CH_3SSS$-W und bezeichnet als Antibiotikum LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E oder CL-1724, worin

   W ist

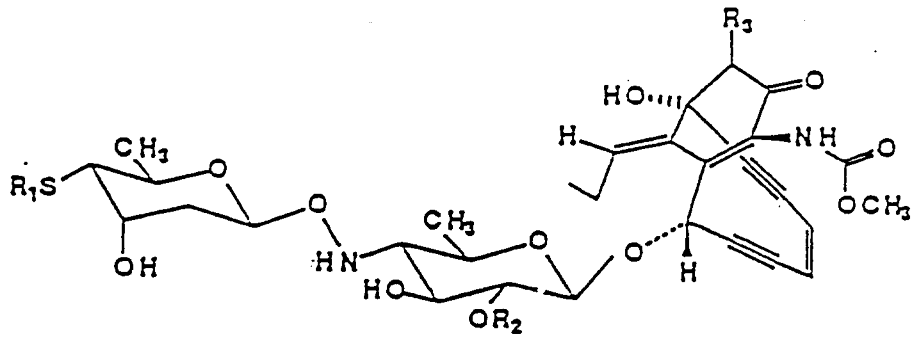

   $R_1$ ist

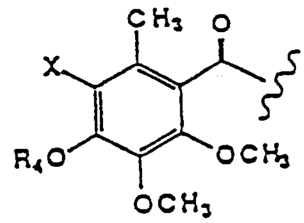

   oder $CH_3$,

   $R_2$ ist

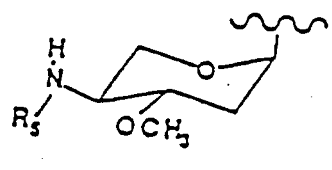

   oder H,

   $R_3$ ist

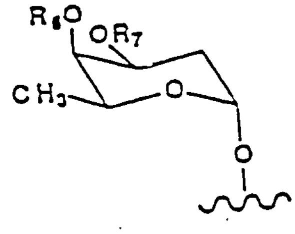

   oder H,

   $R_4$ ist

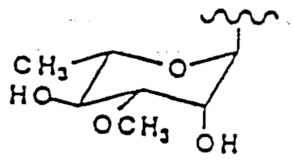

oder H,

$R_5$ ist $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ oder
$CH_3CH_2$

$R_6$ ist

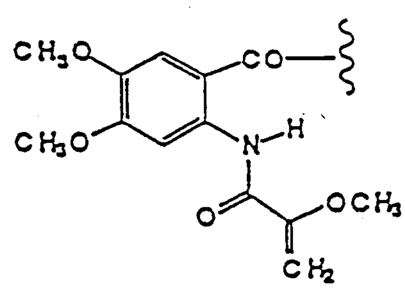

oder H,

$R_7$ ist

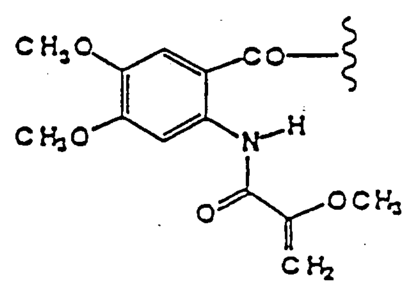

oder H und X ist Jod oder Brom; Sp ist geradkettiger oder verzweigter zweiwertiger $C_1$-$C_{18}$-Rest; zweiwertiger Aryl- oder Heteroarylrest, zweiwertiger $C_3$-$C_{18}$-Cycloalkylrest, zweiwertiger $C_1$-$C_{18}$-Aryl- oder Heteroaryl-Alkylrest, zweiwertiger $C_1$-$C_{18}$-Cycloalkyl-alkylrest oder zweiwertiger ungesättigter $C_2$-$C_{18}$-Alkylrest; Q ist Wasserstoff, Halogen, Amino, Alkylamino, Dialkylamino, Piperidino, Pyrrolidino, Piperazino, Carboxyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$, unter der Bedingung, daß, wenn Sp Ethyliden ist, Q nicht Wasserstoff sein kann, umfassend die Umsetzung eines Alkyltrisulfid-Antibiotikums aus den obigen mit einem geeignet substituierten Mercaptan in Acetonitril bei -10 °C bis -30 °C für 1-48 Stunden.

**2.** Verfahren nach Anspruch 1, das das Antitumormittel LL-E33288$\gamma_2$-I mit

a) einem UV-Spektrum, wie in Figur I gezeigt;
b) einem Protonenmagnetischen Resonanzspektrum, wie in Figur II gezeigt;
c) einem IR-Spektrum, wie in Figur III gezeigt;
d) einem Massenspektrum, wie in Figur IV gezeigt und
e) der folgenden Formel herstellt

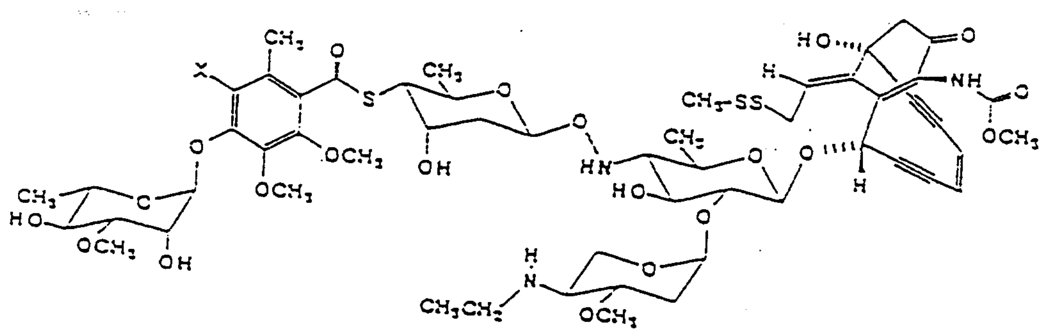

R = $CH_3$, X = J = LL-E33288$\gamma_2^{I}$.

**3.** Verfahren nach Anspruch 1, das das Antitumormittel LL-E33288$\gamma_2$-Br der folgenden Formel herstellt

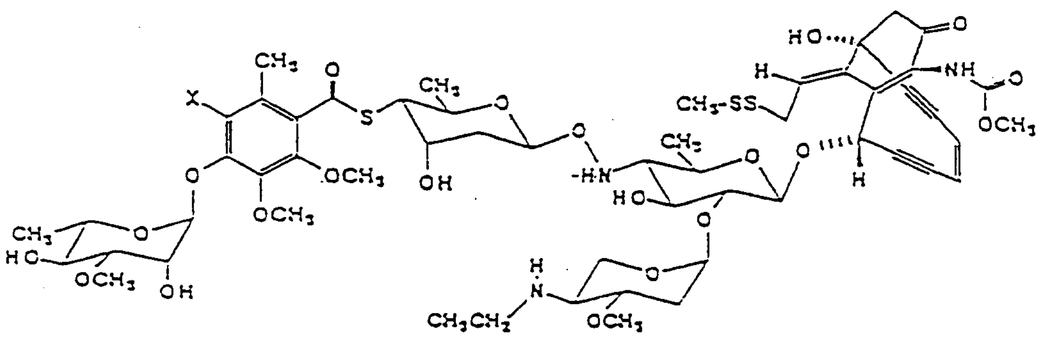

R = $CH_3$, X = Br = LL-E33288$\gamma_2^{Br}$.

**4.** Verfahren nach Anspruch 1, das das Ethyldisulfid-Analoge von LL-E33288$\gamma_1$-I herstellt.

**5.** Verfahren nach Anspruch 1, das das Propyldisulfid-Analoge von LL-E33288$\gamma_1$-I mit einem protonenmagnetischen Resonanzspektrum, wie in Figur V gezeigt und einem Massenspektrum, wie in Figur VI gezeigt, herstellt.

**6.** Verfahren nach Anspruch 1, das das 1-Methyl-1-propyldisulfid-Analoge von LL-E33288$\gamma_1$-I herstellt.

**7.** Verfahren nach Anspruch 1, worin der Heteroalkylrest 7-amino-4-methyl-cumarin ist.

**Revendications**
**Revendications pour les Etats contractants suivants : AT, BE, CH, DE, FR, GB, IT, LI, NL, SE**

**1.** L'agent antitumoral LL-E33288$\gamma_2$-I ayant

a) les spectres dans l'ultraviolet représentés dans la figure I;
b) un spectre de résonance magnétique du proton représenté dans la figure II;
c) un spectre dans l'infrarouge représenté dans la figure III;

d) un spectre de masse représenté dans la figure IV et
e) la formule suivante :

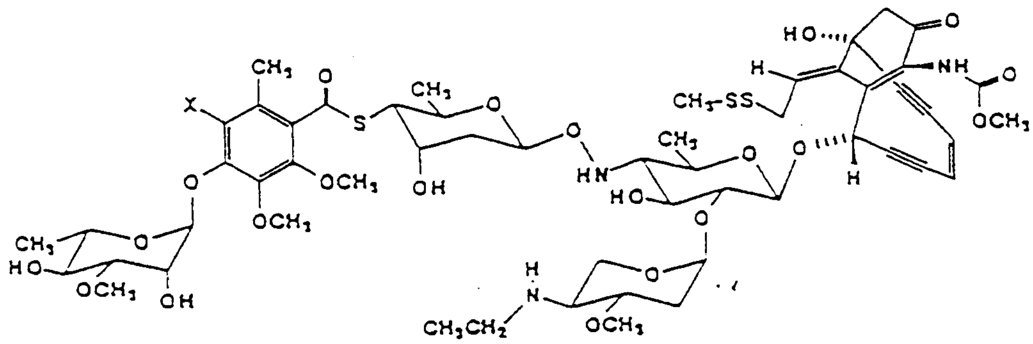

$R = CH_3$, $X = I = LL33288\gamma_2^{I}$

**2.** L'agent antitumoral LL-E33288$\gamma_2$ répondant à la formule :

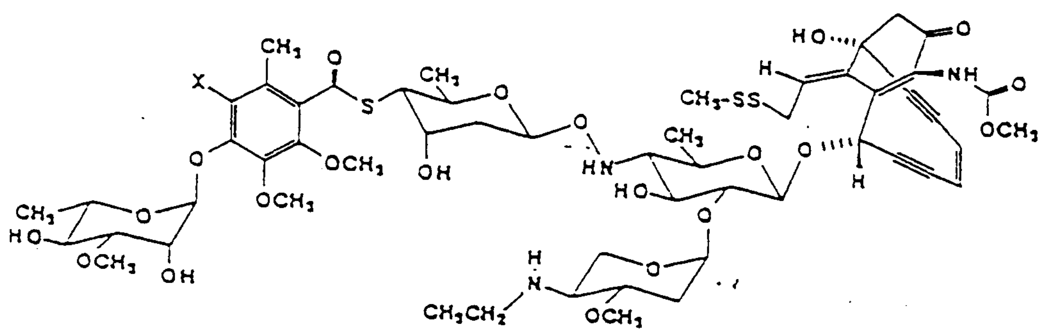

$R = CH_3$, $X = Br = LL33288\gamma_2^{Br}$

**3.** Un disulfure substitué de formule Q-Sp-SS-W, préparé à partir d'un composé de formule $CH_3SSS$-W et dénommé antibiotique LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, Cl-1577B, Cl-1577D, DL-1577E ou CL-1724
dans lequel
W est

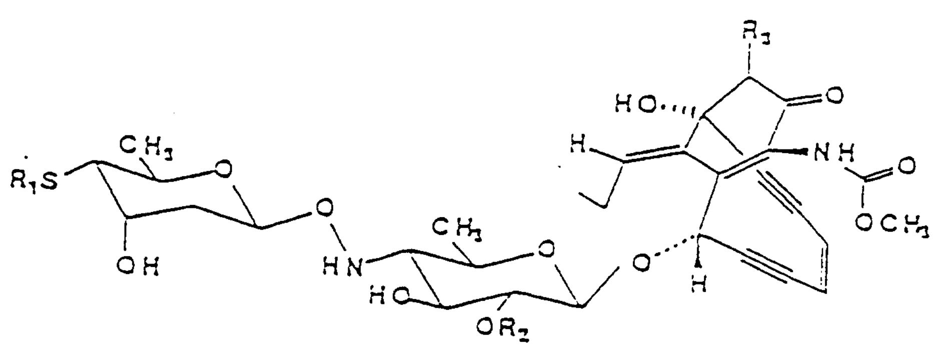

$R_1$ est

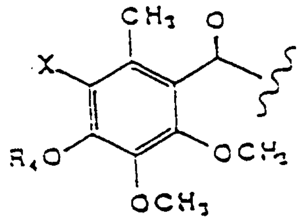

ou $CH_3$,

$R_2$ est

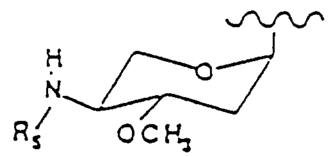

ou H,

$R_3$ est

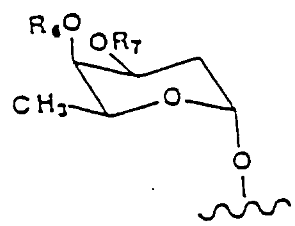

ou H,

$R_4$ est

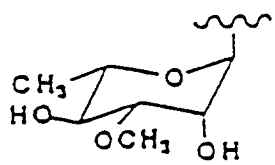

ou H,

$R_5$ est $C_2H_5$
$(CH_3)_2CH$
$C_2H_5$
$CH_3$
$(CH_3)_2CH$
$C_2H_5$
$C_2H_5$

$(CH_3)_2CH$
$(CH_3)_2CH$ ou
$CH_3CH_2$,

$R_6$ est

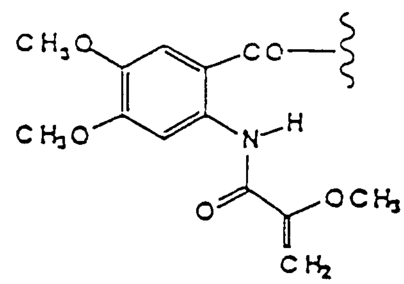

ou H,

$R_7$ est

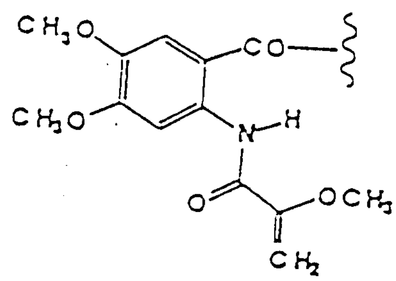

ou H

et X est l'iode ou le brome ; Sp est un radical divalent à chaîne droite ou ramifiée en $C_1$-$C_{18}$ ; un radical aryle ou hétéroaryle divalent, un radical cycloalkyle divalent en $C_3$-$C_{18}$, un radical aryl- ou hétéroaryl-alkyle divalent en $C_1$-$C_{18}$, un radical cycloalkyl-alkyle divalent en $C_1$-$C_{18}$ ou un radical alkyle insaturé divalent en $C_2$-$C_{18}$ ; Q est l'hydrogène ou un halogène ou un groupe amino, alkylamino, dialkylamino, pipéridino, pyrrolidino, pipérazino, carboxyle, carboxaldéhyde, alcoxy inférieur, hydroxy, thiol, alkyldicarboxyle inférieur, $-CONHNH_2$, $-NHCONHNH_2$, $-CSNHNH_2$, $-NHCSNHNH_2$ ou $-ONH_2$; avec la condition que, lorsque Sp est un groupe éthylidène, Q ne peut pas être l'hydrogène.

**4.** L'analogue éthyldisulfure du LL-E33288$\gamma_1$-I.

**5.** L'analogue propyldisulfure du LL-E33288$\gamma_1$-I ayant un spectre de résonance magnétique du proton représenté à la figure V et un spectre de masse représenté à la figure VI.

**6.** L'analogue 1-méthyl-1-propyldisulfure du LL-E33288$\gamma_1$-I.

**7.** Un composé selon la revendication 3, dans lequel le radical hétéroarylalkyle est un reste 7-amino-4-méthylcoumarine.

**8.** Un procédé pour produire le disulfure substitué de formule Q-Sp-SS-W préparé à partir d'un composé de formule $CH_3SSS$-W et dénommé antibiotique LL-E-33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBM-1675, FR-900405, FR-900406, PD 114759, PD 115028, CL-1577A, CL-1577B, CL-1577D, CL-1577E ou CL-1724
dans lequel
W est

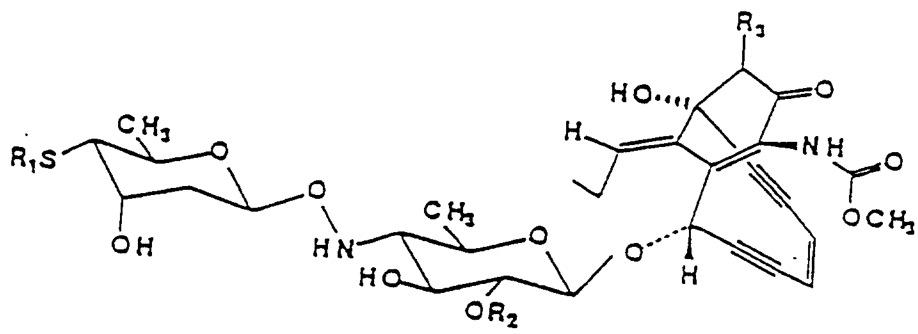

$R_1$ est

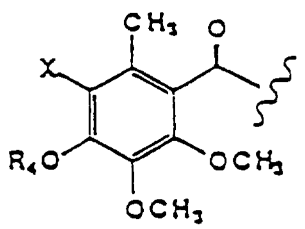

ou $CH_3$,

$R_2$ est

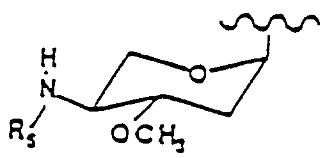

ou H,

$R_3$ est

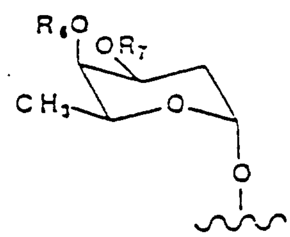

ou H,

$R_4$ est

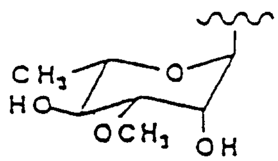

ou H,

$R_5$ est $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ ou
$CH_3CH_2$,

$R_6$ est

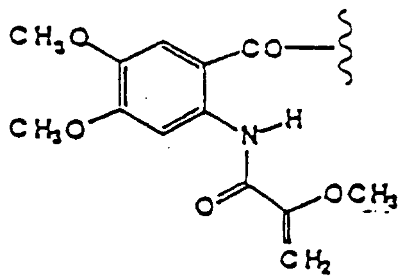

ou H,

$R_7$ est

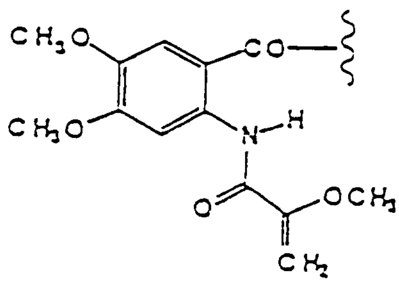

ou H

et X est l'iode ou le brome ; Sp est un radical divalent à chaîne droite ou ramifiée en $C_1$-$C_{18}$, un radical aryle ou hétéroaryle divalent, un radical cycloalkyle divalent en $C_3$-$C_{18}$, un radical aryl- ou hétéroaryl-alkyle divalent en $C_1$-$C_{18}$, un radical cycloalkyl-alkyle divalent en $C_1$-$C_{18}$ ou un radical alkyle insaturé divalent en $C_2$-$C_{18}$ ; Q est l'hydrogène ou un halogène ou un groupe amino, alkylamino, dialkylamino, pipéridino, pyrrolidino, pipérazino, carboxyle, carboxaldéhyde, alcoxy inférieur, hydroxy, thiol, alkyldicarboxyle inférieur, $-CONHNH_2$, $-NHCONHNH_2$, $-CSNHNH_2$, $-NHCSNHNH_2$ ou $-ONH_2$ ; avec la condition que, lorsque Sp est un groupe éthylidène, Q ne peut pas être l'hydrogène ; qui comprend la réaction d'un alkyltrisulfure antibiotique parmi ceux cités ci-dessus avec un mercaptan substitué approprié dans l'acétonitrile à une température de -10 à -30 °C pendant 1-48 h.

9. L'utilisation d'un disulfure substitué de formule Q-Sp-SS-W, préparé à partir d'un composé de formule $CH_3SSS$-W et dénommé antibiotique LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D ou CL-1577E,
dans lequel
W est

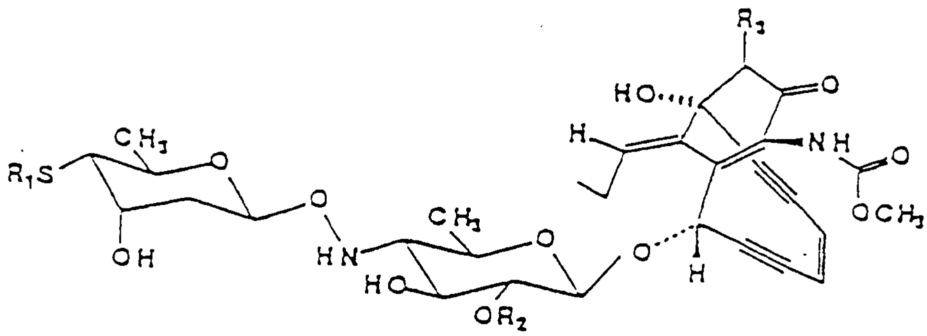

$R_1$ est

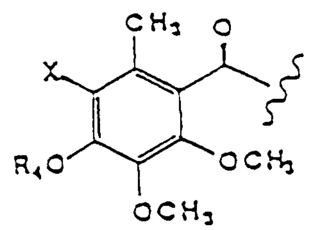

ou $CH_3$,
$R_2$ est

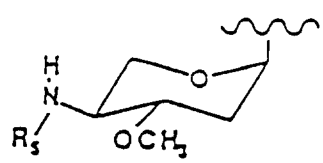

ou H,
$R_3$ est

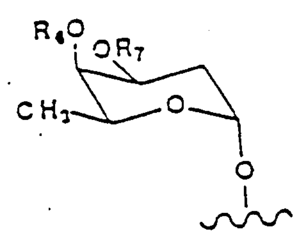

ou H,

$R_4$ est

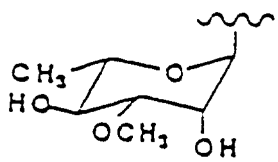

ou H,

$R_5$ est $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ ou
$CH_3CH_2$,

$R_6$ est

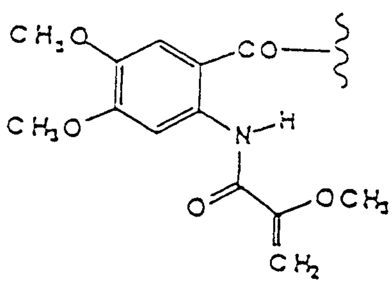

ou H,

$R_7$ est

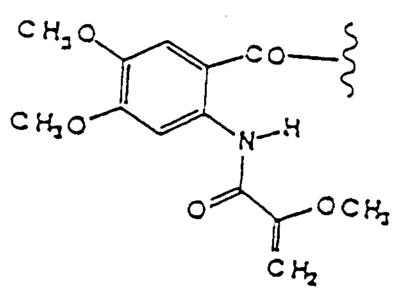

ou H

et X est l'iode ou le brome ; Sp est un radical divalent à chaîne droite ou ramifiée en $C_1$-$C_{18}$, un radical aryle ou hétéroaryle divalent, un radical cycloalkyle divalent en $C_3$-$C_{18}$, un radical aryl- ou hétéroaryl-

alkyle divalent en $C_1$-$C_{18}$, un radical cycloalkylalkyle divalent en $C_1$-$C_{18}$ ou un radical alkyle divalent insaturé en $C_2$-$C_{18}$ ; Q est l'hydrogène ou un halogène ou un groupe amino, alkylamino, dialkylamino, pipéridino, pyrrolidino, pipérazino, carboxyle, carboxaldéhyde, alcoxy inférieur, hydroxy, thiol, alkyldicarboxyle inférieur, $-CONHNH_2$, $-NHCONHNH_2$, $-CSNHNH_2$, $-NHCSNHNH_2$, ou $-ONH_2$ ; avec la condition que, lorsque Sp est un groupe éthylidène, Q ne peut pas être l'hydrogène, pour la fabrication d'un médicament pour traiter les infections bactériennes chez les animaux à sang chaud.

10. L'utilisation d'un disulfure substitué de formule Q-Sp-SS-W, préparé à partir d'un composé de formule $CH_3SSS$-W et dénommé antibiotique LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBM-1675, FR-900405, FR-900406, PD 114759, PD 115028, CL-1577A, CL-1577B, CL1577D, CL-1577E ou CL-1724

dans lequel

W est

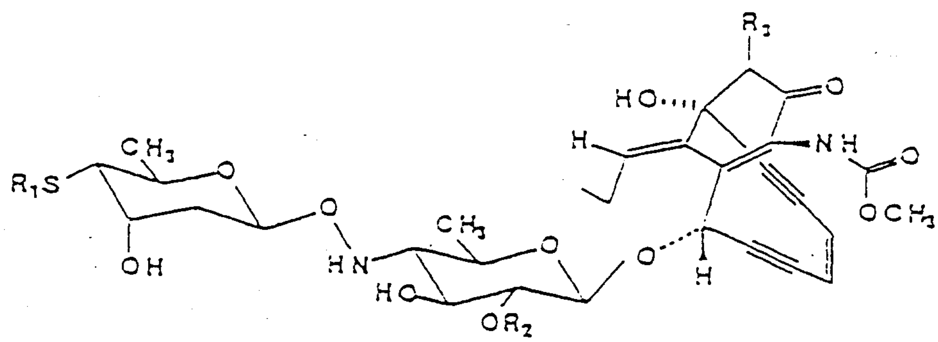

R₁ est

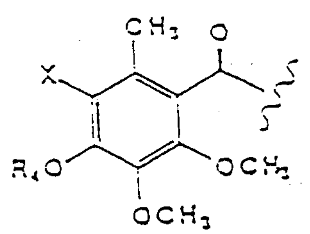

ou $CH_3$,

$R_2$ est

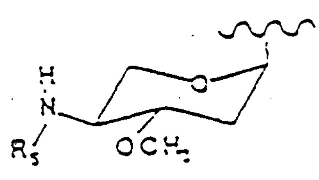

ou H,

$R_3$ est

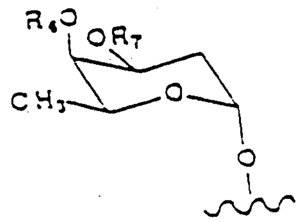

ou H,

$R_4$ est

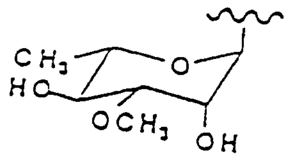

ou H,

$R_3$ est $C_2H_5$

$(CH_3)_2CH$

$C_2H_5$

$CH_3$

$(CH_3)_2CH$

$C_2H_5$

$C_2H_5$

$(CH_3)_2CH$

$(CH_3)_2CH$ ou

$CH_3CH_2$,

$R_6$ est

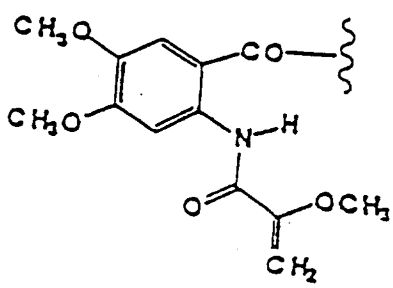

ou H,

$R_7$ est

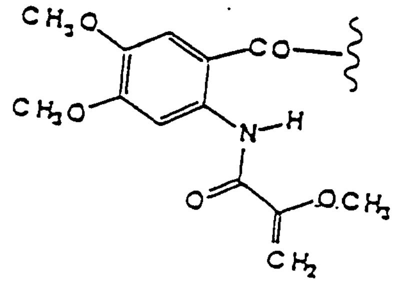

ou H

et X est l'iode ou le brome ; Sp est un radical divalent à chaîne droite ou ramifiée en $C_1$-$C_{18}$, un radical aryle ou hétéroaryle divalent, un radical cycloalkyle divalent en $C_3$-$C_{18}$, un radical aryl- ou hétéroaryl-alkyle divalent en $C_1$-$C_{18}$, un radical cycloalkylalkyle divalent en $C_1$-$C_{18}$ ou un radical alkyle divalent insaturé en $C_2$-$C_{18}$ ; Q est l'hydrogène ou un halogène ou un groupe amino, alkylamino, dialkylamino, pipéridino, pyrrolidino, pipérazino, carboxyle, carboxaldéhyde, alcoxy inférieur, hydroxy, thiol, alkyldicarboxyle inférieur, ou un groupe choisi parmi les suivants : $-CONHNH_2$, $-NHCONHNH_2$, $-CSNHNH_2$, $-NHCSNHNH_2$, ou $-ONH_2$ ; avec la condition que, lorsque Sp est un groupe éthylidène, Q ne peut pas être l'hydrogène, pour la fabrication d'un médicament pour inhiber la croissance des tumeurs chez un mammifère.

**Revendications pour les Etats contractants suivants : ES, GR**

**1.** Un procédé pour produire le disulfure substitué de formule Q-Sp-SS-W, préparé à partir d'un composé de formule $CH_3SSS$-W et dénommé antibiotique LL-E33288$\alpha_1$-Br, $\alpha_1$-I, $\alpha_2$-Br, $\alpha_2$-I, $\alpha_3$-Br, $\alpha_3$-I, $\alpha_4$-Br, $\beta_1$-Br, $\beta_1$-I, $\beta_2$-I, $\gamma_1$-Br, $\gamma_1$-I, $\delta_1$-I, BBm-1675, FR-900405, FR-900406, PD 114759, PD 115028, CL-1577A, CL-1577B, CL-1577D, CL-1577E ou CL-1724,
dans lequel
W est

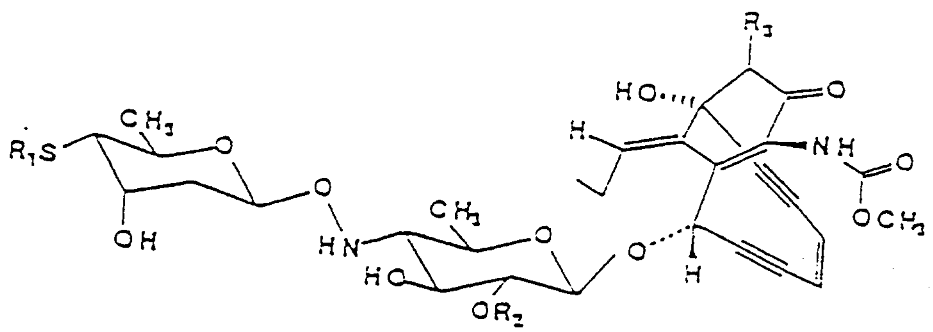

$R_1$ est

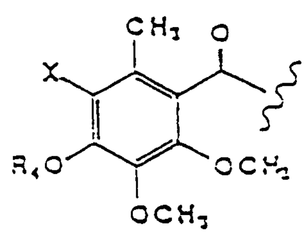

ou $CH_3$,

$R_2$ est

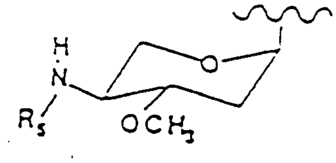

ou H,

$R_3$ est

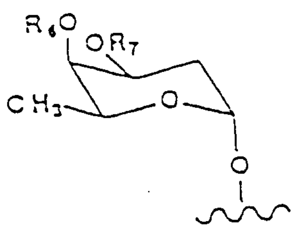

ou H,

$R_4$ est

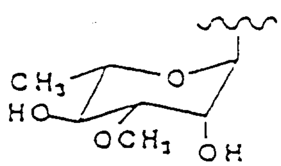

ou H,

$R_5$ est $C_2H_5$
$(CH_3)_2CH$
$C_2H_5$
$CH_3$
$(CH_3)_2CH$
$C_2H_5$
$C_2H_5$
$(CH_3)_2CH$
$(CH_3)_2CH$ ou
$CH_3CH_2$,

$R_6$ est

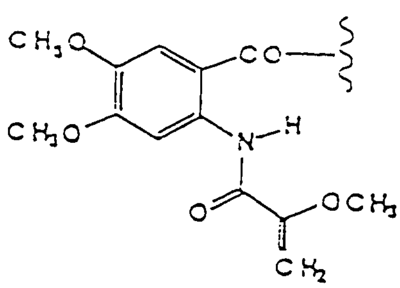

ou H,

$R_7$ est

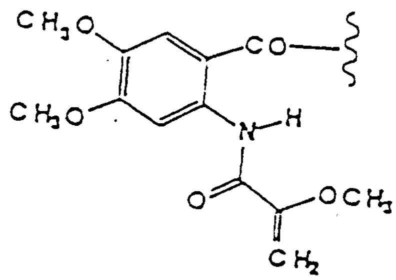

ou H

et X est l'iode ou le brome ; Sp est un radical divalent à chaîne droite ou ramifiée en $C_1$-$C_{18}$ ; un radical aryle ou hétéroaryle divalent, un radical cycloalkyle divalent en $C_3$-$C_{18}$, un radical aryl- ou hétéroaryl-alkyle divalent en $C_1$-$C_{18}$, un radical cycloalkyl-alkyle divalent en $C_1$-$C_{18}$ ou un radical alkyle insaturé divalent en $C_2$-$C_{18}$ ; Q est l'hydrogène ou un halogène ou un groupe amino, alkylamino, dialkylamino, pipéridino, pyrrolidino, pipérazino, carboxyle, carboxaldéhyde, alcoxy inférieur, hydroxy, thiol, alkyldicarboxyle inférieur, $-CONHNH_2$, $-NHCONHNH_2$, $-CSNHNH_2$, $-NHCSNHNH_2$ ou $-ONH_2$ ; avec la condition que, lorsque Sp est un groupe éthylidène, Q ne peut pas être l'hydrogène; qui comprend la réaction d'un alkyltrisulfure antibiotique parmi ceux cités ci-dessus avec un mercaptan substitué approprié dans l'acétonitrile à une température de -10 à -30 ° C pendant 1-48 h.

**2.** Le procédé selon la revendication 1 qui produit l'agent antitumoral LL-E33288$\gamma_2$-I ayant
   a) les spectres dans l'ultraviolet représentés dans la figure I;
   b) un spectre de résonance magnétique du proton représenté dans la figure II;
   c) un spectre dans l'infrarouge représenté dans la figure III;
   d) un spectre de masse représenté dans la figure IV et
   e) la formule suivante :

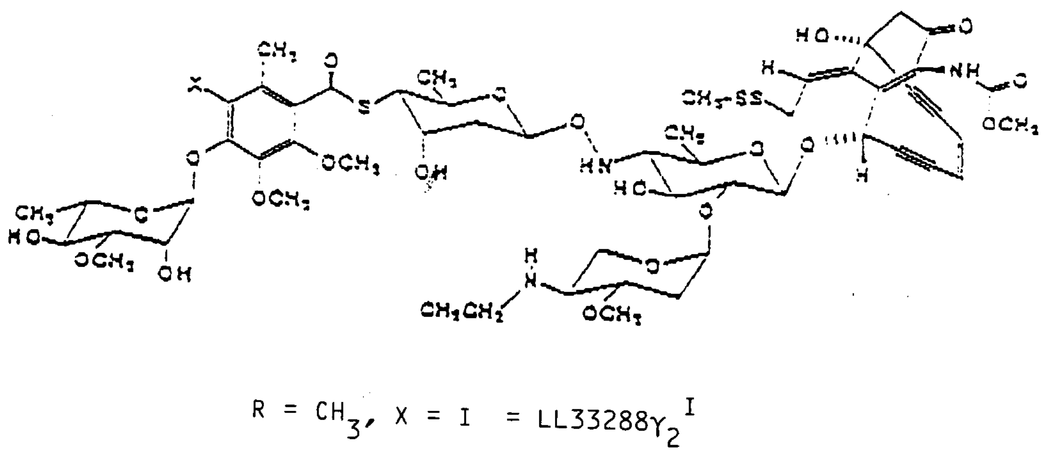

R = $CH_3$, X = I = LL33288$\gamma_2^{I}$

**3.** Le procédé selon la revendication 1, qui produit l'agent antitumoral LL-E33288$\gamma_2$-Br répondant à la formule suivante :

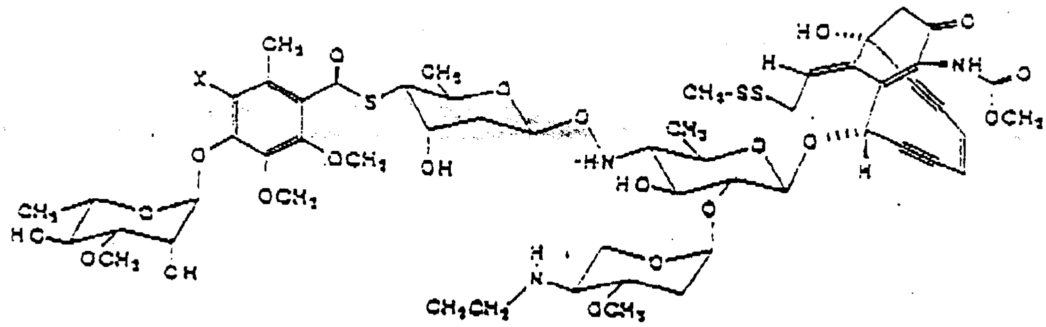

R = $CH_3$, X = Br = LL33288$\gamma_2^{Br}$

**4.** Le procédé selon la revendication 1, qui produit l'analogue éthyldisulfure du LL-E33288$\gamma_1$-I.

**5.** Le procédé selon la revendication 1, qui produit l'analogue propyldisulfure du LL-E33288$\gamma_1$-I ayant un spectre de résonance magnétique du proton représenté à la figure V et un spectre de masse représenté à la figure VI.

**6.** Le procédé selon la revendication 1, qui produit l'analogue 1-méthyl-1-propyldisulfure du LL-E33288$\gamma_1$-I.

**7.** Le procédé selon la revendication 1, dans lequel le radical hétéroarylalkyle est un reste 7-amino-4-méthylcoumarine.

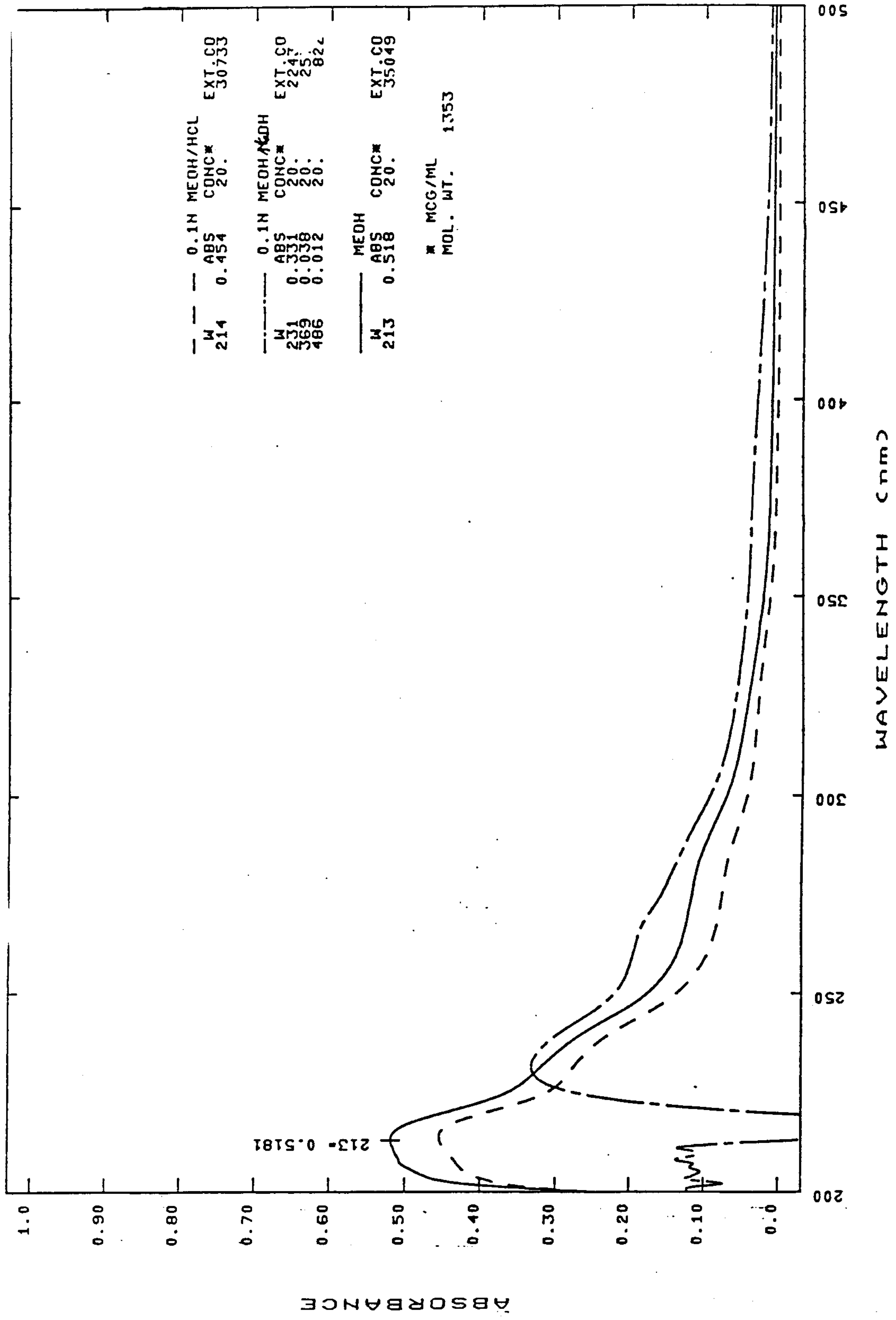
0.1N MEOH/HCL
W 214
ABS 0.454
CONC* 20.
EXT.CO 30733
W 231 369 486
ABS 0.331 0.038 0.012
CONC* 20. 20. 20.
MEOH
W 213
ABS 0.518
CONC* 20.
EXT.CO 35049
* MCG/ML
MOL. WT. 1353
213- 0.5181
ABSORBANCE
WAVELENGTH (nm)
1.0
0.90
0.80
0.70
0.60
0.50
0.40
0.30
0.20
0.10
0.0
200
250
300
350
400
450
500

FIGURE I

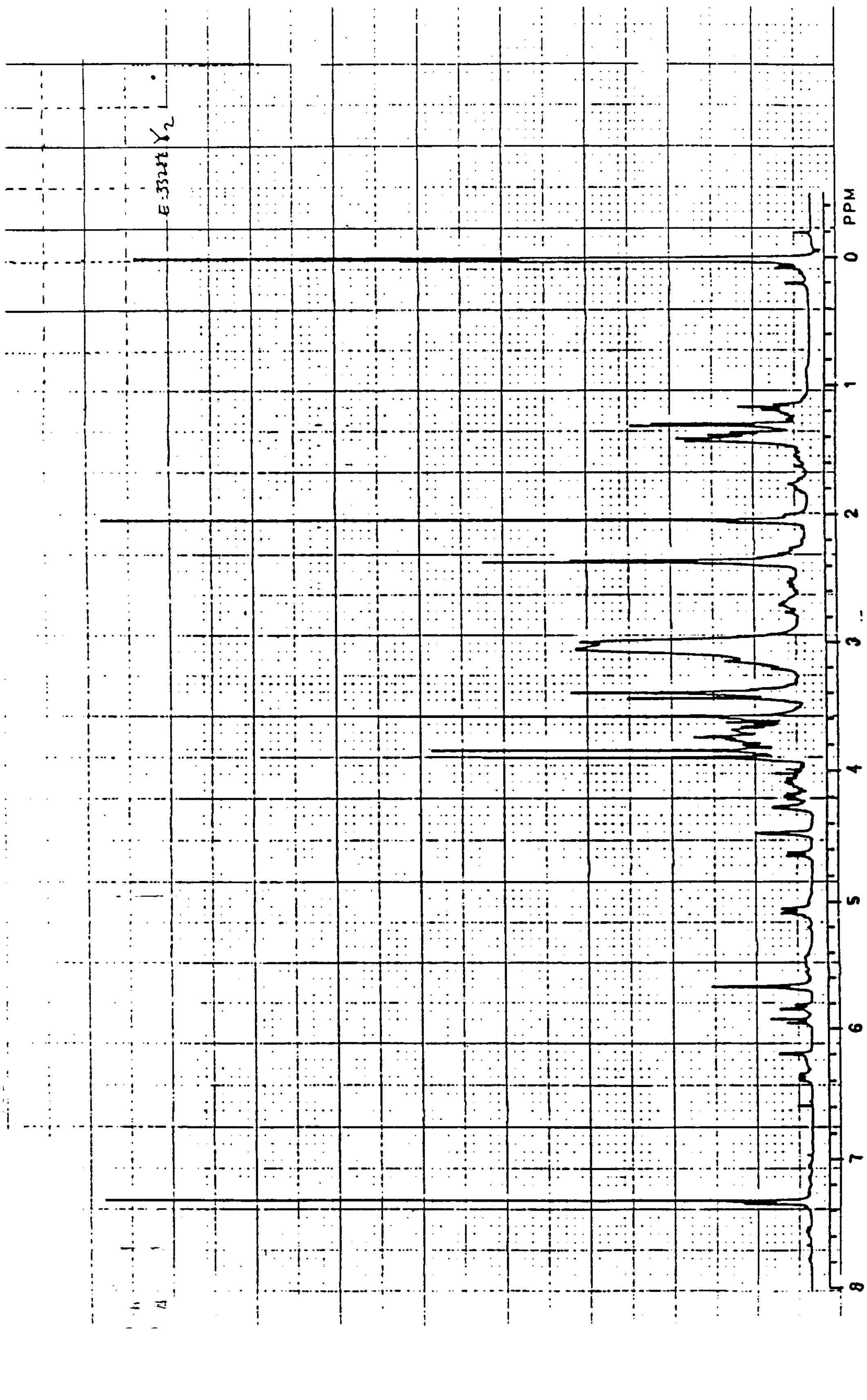


FIGURE II

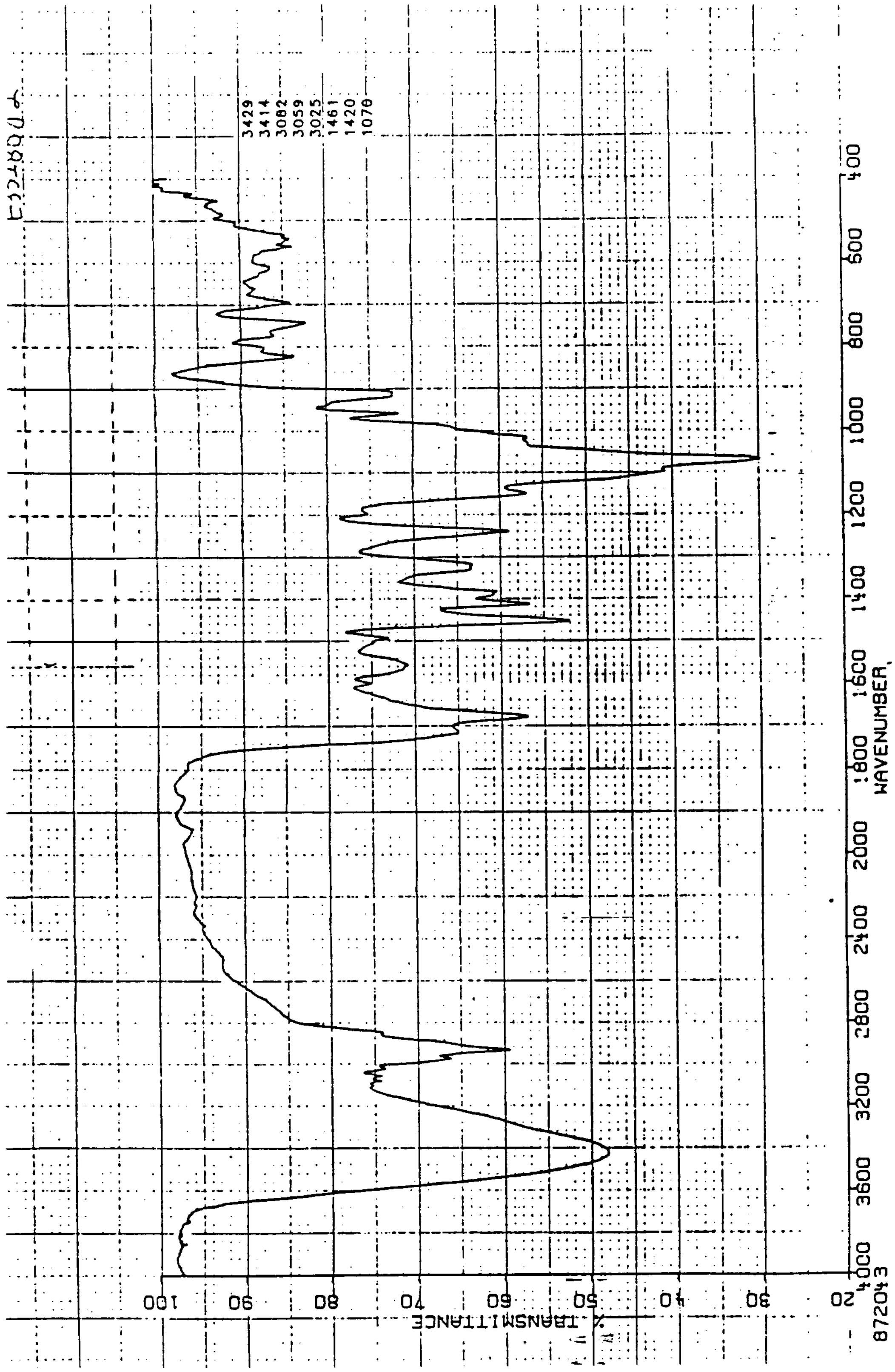
3429
3414
3082
3059
3025
1461
1420
1070
4000
3600
3200
2800
2400
2000
1800
1600
1400
1200
1000
800
600
400
WAVENUMBER
% TRANSMITTANCE
100
90
80
70
60
50
40
30
20
872043

FIGURE III

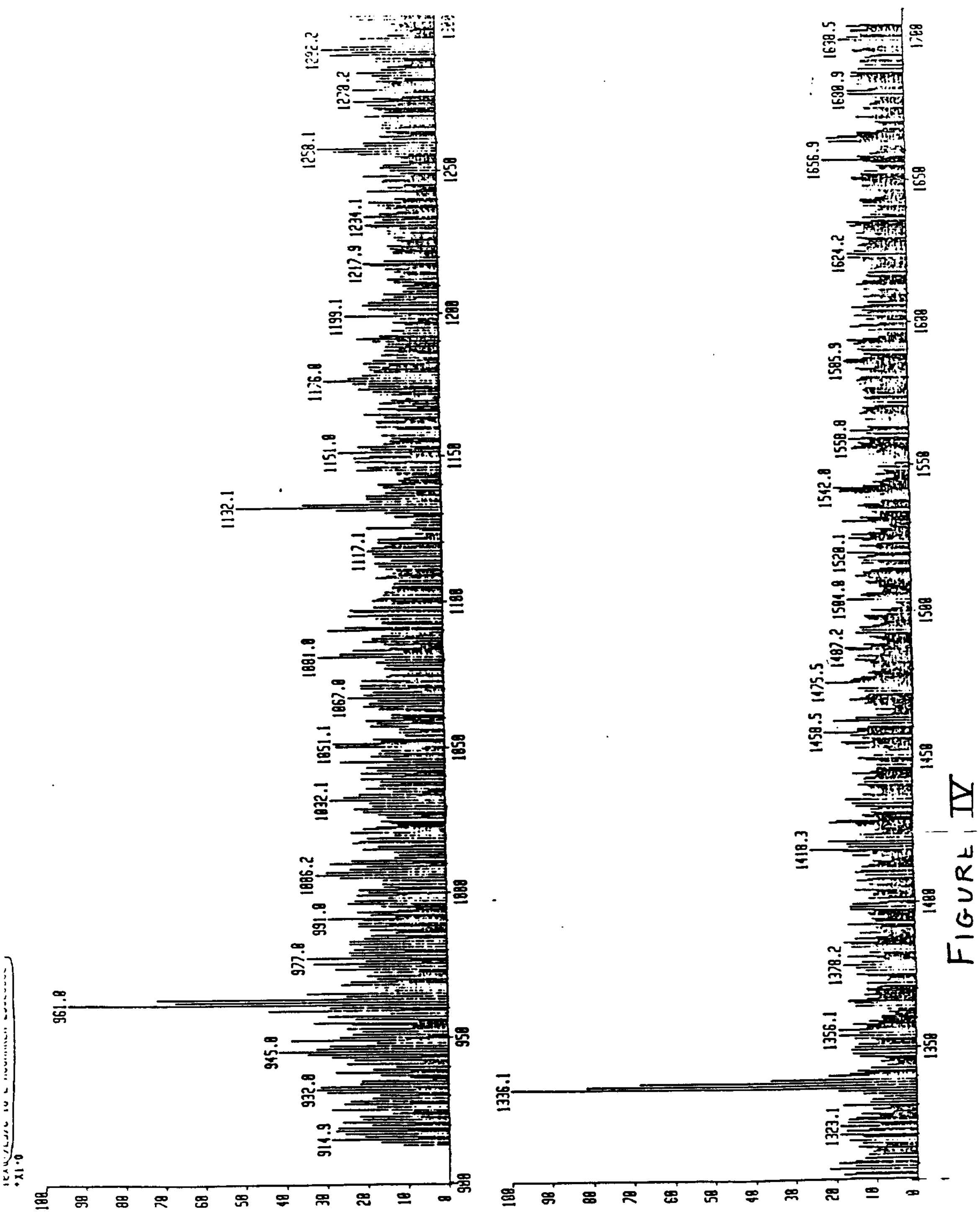
914.9
932.0
945.0
961.0
977.0
991.0
1006.2
1032.1
1051.1
1067.0
1081.0
1117.1
1132.1
1151.0
1176.0
1199.1
1217.9
1234.1
1258.1
1278.2
1323.1
1336.1
1356.1
1378.2
1418.3
1458.5
1475.5
1487.2
1504.0
1520.1
1542.0
1558.0
1585.9
1624.2
1656.9
1680.9
1690.5

FIGURE IV

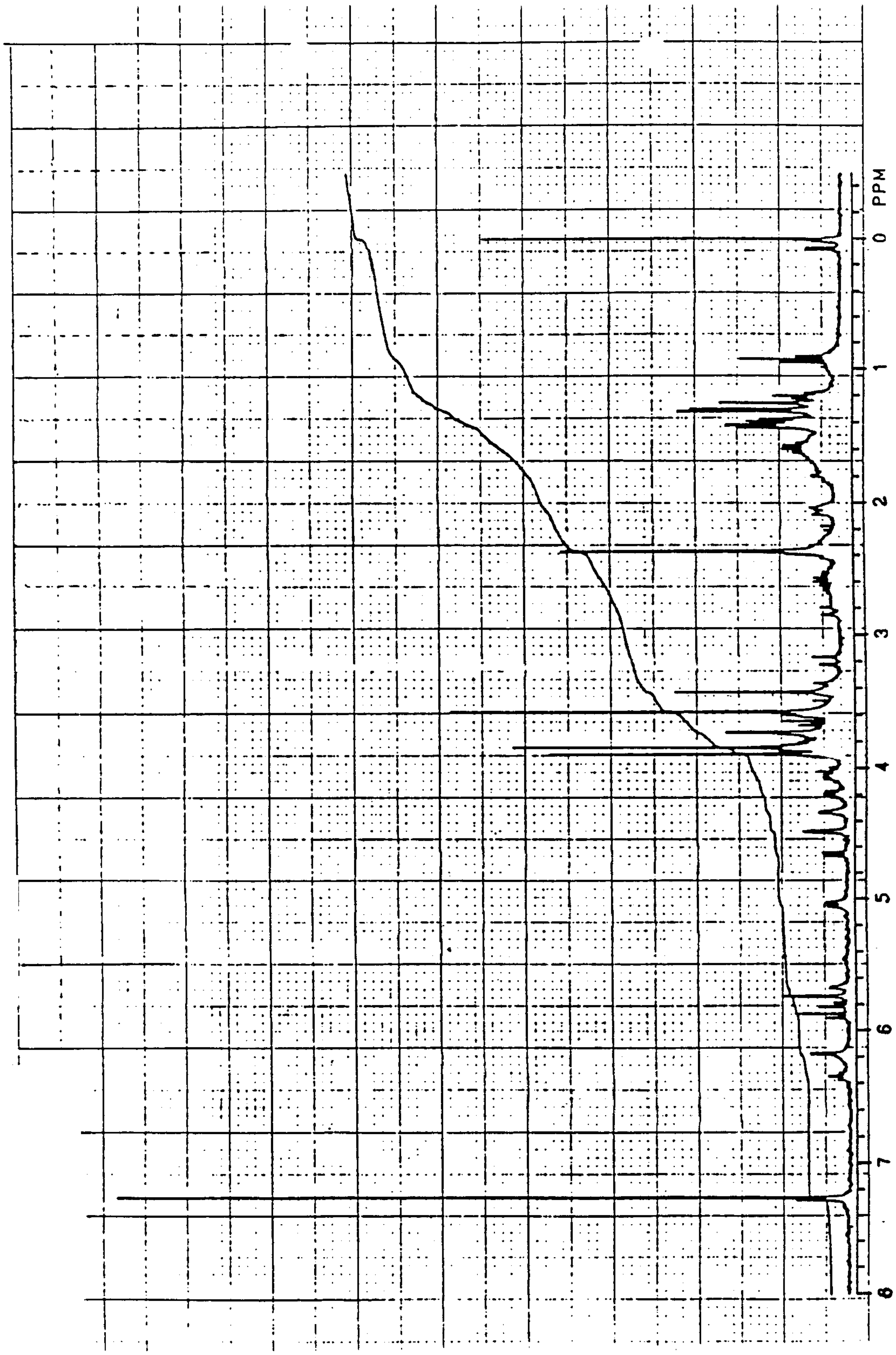


FIGURE V

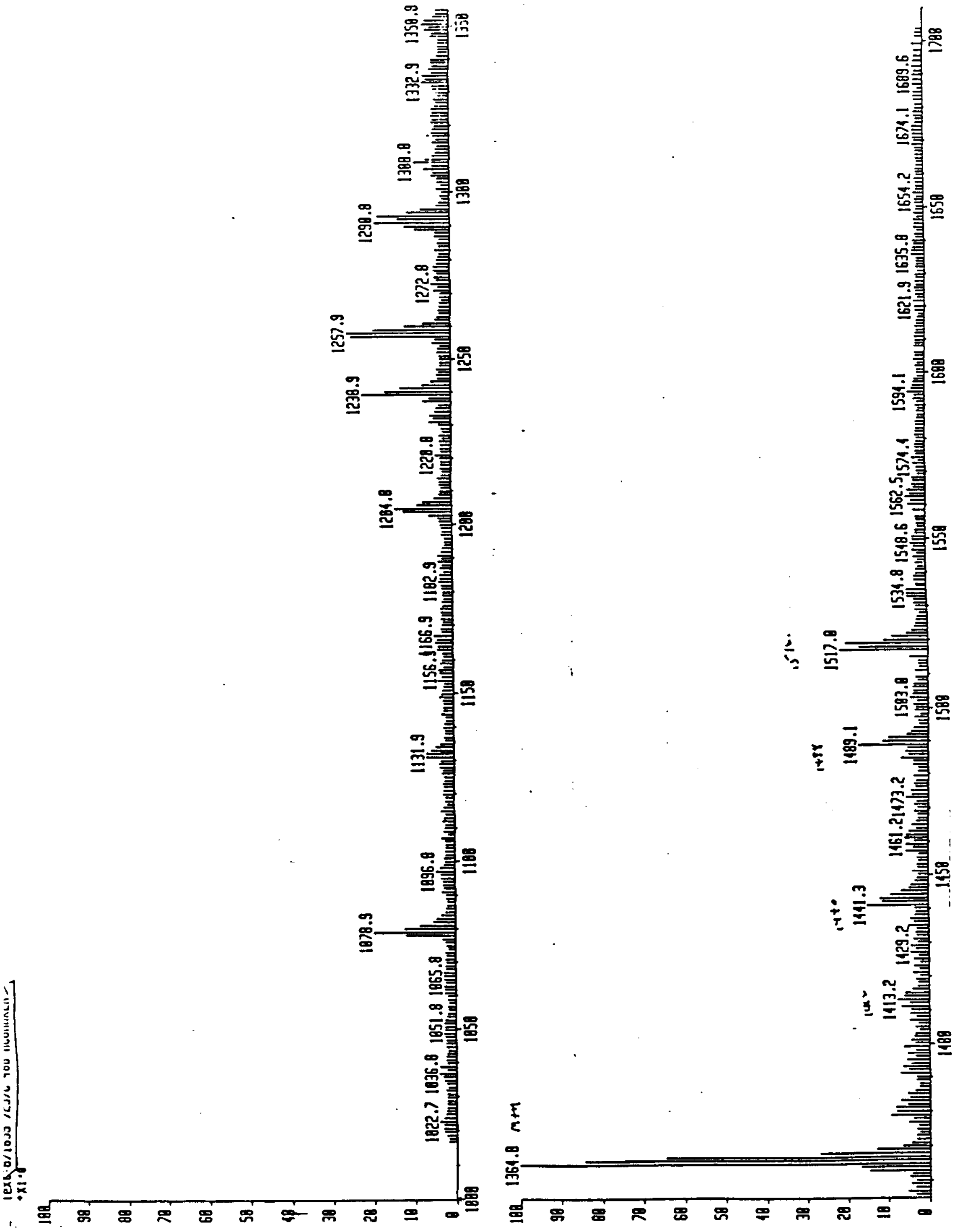
1022.7
1036.8
1051.8
1065.8
1078.9
1096.8
1131.9
1156.9
1166.9
1182.9
1204.8
1220.8
1238.9
1257.9
1272.8
1290.8
1308.8
1332.9
1350.9
1364.8
1413.2
1429.2
1441.3
1461.2
1473.2
1489.1
1503.8
1517.8
1534.8
1548.6
1562.5
1574.4
1594.1
1621.9
1635.8
1654.2
1674.1
1689.6

FIGURE VI